# EUROPEAN PATENT SPECIFICATION

(11) **EP 1 568 317 B1**
(45) Date of publication and mention of the grant of the patent: **06.06.2012**
(21) Application number: 03774162.6
(22) Date of filing: 21.11.2003
(51) Int. Cl.: A61B 5/02

(54) **PULSE MEASURING INSTRUMENT**
PULSMESSINSTRUMENT
INSTRUMENT DE MESURE DU POULS

(30) Priority: 29.11.2002 JP 2002347624; 21.10.2003 JP 2003360724
(43) Date of publication of application: 31.08.2005
(73) Proprietor: Omron Healthcare Co., Ltd., Kyoto 615-0084 (JP)
(72) Inventor: ITONAGA, Kazunobu, c/o OMRON Healthcare Co., Ltd., Kyoto-shi, Kyoto 615-0084 (JP); TANABE, Kazuhisa, c/o OMRON Healthcare Co., Ltd., Kyoto-shi, Kyoto 615-0084 (JP); INAGAKI, Takashi, c/o OMRON Healthcare Co., Ltd., Kyoto-shi, Kyoto 615-0084 (JP)
(74) Representative: Kilian, Helmut
(86) International application number: PCT/JP2003/014950
(87) International publication number: WO 2004/049933

(56) References cited:
- JP-A- 4 067 839
- JP-A- 7 116 136
- JP-A- 8 103 419
- US-A- 5 176 143
- US-A- 5 176 143
- US-A- 5 467 771
- US-A1- 2002 026 121
- US-B1- 6 331 161
- US-B1- 6 428 476

## Description

### Technical Field

The present invention relates to a pulse wave measuring apparatus, and more particularly to a pressurization-type pulse wave measuring apparatus measuring a pulse wave by pressing a substrate including pressure-sensing means against a living body.

### Background Art

In general, a pressurization-type pressure measuring apparatus for measuring contact pressure with respect to a target object by pressing the apparatus against the target object has been known. An application example of such a pressurization-type pressure measuring apparatus includes a pulse wave measuring apparatus. A pulse wave measuring apparatus is used for measuring a pulse wave by pressing a substrate having pressure-sensing means against a body surface in order to measure a pulse wave generated in an artery located in a relatively shallow portion under the skin of the living body. In order to know medical condition of a subject, it is extremely important to measure a pulse wave of the subject, using such a pulse wave measuring apparatus.

In the pressurization-type pulse wave measuring apparatus, a semiconductor pressure sensor utilizing a strain gauge or a diaphragm is commonly used as the pressure-sensing means. In such a case, the substrate is arranged such that the pressure-sensing means for detecting the pulse wave is located on a surface of a housing attached to the living body. For example, Japanese Patent Laying-Open No. 4-67839 relates to such a pressurization-type pulse wave measuring apparatus.

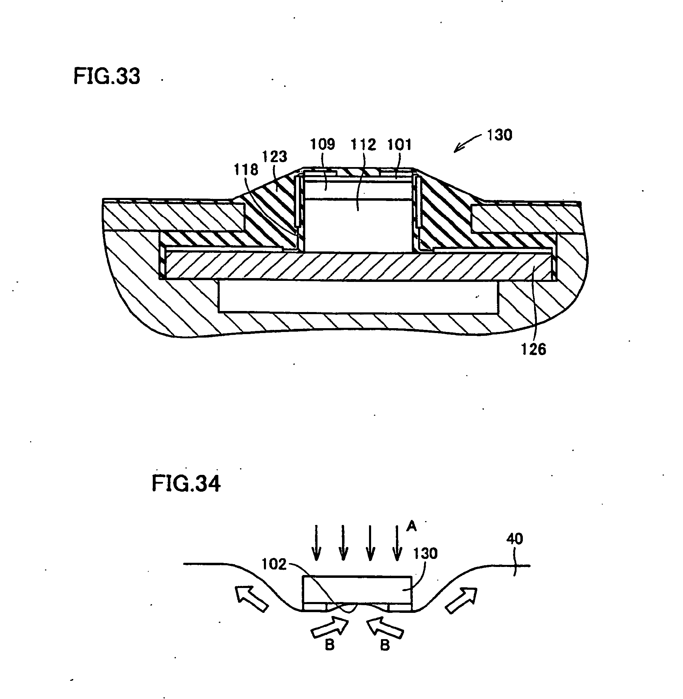

Fig. 33 is a schematic partial cross-sectional view of the pulse wave measuring apparatus disclosed in the above publication. As shown in Fig. 33, the pulse wave measuring apparatus disclosed in this publication includes a pressure-sensing portion 130 on the surface of the housing. Pressure-sensing portion 130 includes a semiconductor substrate 101 having a pressure-sensing device formed on a main surface, a support member 109 supporting semiconductor substrate 101, and a protection member 112 fixing support member 109. A circuit board 126 provided with a processing circuit processing a signal output from the pressure-sensing device is arranged in the housing. A flexible line 118 establishes electrical connection between semiconductor substrate 101 having the pressure-sensing device formed and circuit board 126. In order to protect the pressure-sensing device, pressure-sensing portion 130 is sealed by silicon rubber 123. In other words, silicon rubber 123 covers an upper surface and an end surface of semiconductor substrate 101 having the pressure-sensing device formed.

On the other hand, the pulse wave measuring apparatus of a structure described above suffers from a variety of problems as below.

First, volume fluctuation may take place in the silicon rubber covering a periphery of the semiconductor substrate due to variation in an ambient temperature and heat transfer from the body surface. As the volume fluctuation acts as a stress on the semiconductor substrate, there is a possibility that the stress acts on the pressure-sensing device and that resultant noise is superposed on the detected pulse wave. Such volume fluctuation of the silicon rubber occurs also by absorption by the silicon rubber of perspiration on the body surface of the subject. In addition, if a void is present in the silicon rubber or between the semiconductor substrate and the silicon rubber, volume fluctuation of the void itself is further produced in addition to the volume fluctuation of the silicon rubber, whereby the stress is applied to the semiconductor substrate in a complicated manner. Consequently, accurate measurement of the pulse wave becomes more difficult.

Secondly, deformation of the semiconductor substrate tends to be suppressed by the silicon rubber. A force due to a pressure produced in accordance with pulsation is applied to the semiconductor substrate in a direction orthogonal to a pressure-sensing surface. When such a force is applied, the semiconductor substrate attempts to deform slightly so as to extend in a lateral direction. On the other hand, as the end surface of the semiconductor substrate is sealed by the silicon rubber in the structure above, deformation in the lateral direction of the semiconductor substrate is suppressed. Accordingly, stress distribution in the semiconductor substrate becomes complicated, and resultant noise may be superposed on the pulse wave detected by the pressure-sensing device.

Thirdly, skin tension may be applied to the pressure-sensing surface. In the following, this problem will be described in detail with reference to the drawing.

Fig. 34 is a schematic diagram pointing out the problem in the conventional pulse wave measuring apparatus. As shown in Fig. 34, in the pressurization-type pulse wave measuring apparatus, pressure-sensing portion 130 is pressed against a body surface 40 (in a direction shown with an arrow A in the figure) so as to measure the pulse wave. When a pressure-sensing surface 102 is flat, the skin tension acts in a direction parallel to the pressure-sensing surface, and therefore, the skin tension does not affect the pressure-sensing device.

In the pulse wave measuring apparatus disclosed in the publication above, however, as shown in Fig. 33, flexible line 118 for transmitting a signal output from the pressure-sensing device to circuit board 126 is connected to the main surface of semiconductor substrate 101. Accordingly, on the main surface of semiconductor substrate 101, there exist a region carrying silicon rubber 123 alone and a region carrying both silicon rubber 123 and flexible line 118.

Flexible line 118 has elasticity significantly poorer than silicon rubber 123. Therefore, in the pulse wave measuring apparatus disclosed in the publication above, as shown in Fig. 34, such poor elasticity is comparable to a condition in which the pressure-sensing surface has irregularities. Here, as shown in Fig. 34, the skin tension is applied in a direction orthogonal to pressure-sensing surface 102 (in a direction shown with an arrow B in the figure) to the skin directly under pressure-sensing surface 102. As a result, a component of force of the skin tension acts on pressure-sensing surface 102. Then, stress distribution in semiconductor substrate 101 becomes complicated, and resultant noise may be superposed on the detected pulse wave.

As described above, in the pulse wave measuring apparatus disclosed in the publication above, a variety of stresses act on the semiconductor substrate, and the resultant noise is superposed on the detected pulse wave. Consequently, it has been difficult to measured the pulse wave with high accuracy in a stable manner.

On the other hand, in order to achieve sufficient protection effect of the silicon rubber against the stress from a side surface to the semiconductor substrate, the silicon rubber for sealing should have a sufficiently large thickness. Larger thickness of the silicon rubber, however, leads to a larger size of the pulse wave measuring apparatus, which results in difficulty in achieving a shape fitting to the living body.

As described above, in the pulse wave measuring apparatus disclosed in the publication above, the pressure-sensing portion is disadvantageously made larger in order to ensure an effect to protect the pressure-sensing device, and it has been difficult to provide a compact and high-performance pulse wave measuring apparatus.

A pulse wave measuring apparatus according to the preamble of claim 1 is known from US 5 176 143 A1 and US 5 467 771 A1.

### Disclosure of the Invention

An object of the present invention is to provide a pulse wave measuring apparatus capable of accurate and stable measurement of a pulse wave. In addition, it is another object of the present invention to provide a compact and high-performance pulse wave measuring apparatus.

A pulse wave measuring apparatus according to the present invention is as defined in claim 1. It includes a substrate having pressure-sensing means on a main surface, and a protection member having an accommodation space accommodating the substrate. The pulse wave measuring apparatus serves to measure a pulse wave by pressing the substrate against a living body. In the pulse wave measuring apparatus according to the first aspect of the present invention, a wall surface of the protection member forming the accommodation space is arranged such that an air chamber is interposed between the wall surface and an end surface of the substrate.

In this manner, the wall surface of the protection member forming the accommodation space and the end surface of the substrate are arranged spaced apart from each other, so that the end surface of the substrate having the pressure-sensing means is surrounded by the air chamber. As such, even if variation in the ambient temperature or heat transfer from the body surface takes place, stress distribution in the substrate will not be complicated. In other words, as the end surface of the substrate is surrounded by the air chamber, stress caused by volume fluctuation of another member when the end surface of the substrate is covered by another member will not be applied to the substrate, whereby accurate and stable measurement of the pulse wave is allowed.

In addition, as the end surface of the substrate is surrounded by the air chamber, deformation of the substrate in the lateral direction caused by pressurization of the substrate against the subject is no longer suppressed. Accordingly, stress distribution in the substrate will not be complicated, and consequently, accurate and stable measurement of the pulse wave is allowed.

Moreover, by disposing the substrate in the accommodation space in the protection member, protection of the substrate by the protection member is ensured. Therefore, a compact and high-performance pulse wave measuring apparatus can be provided.

In the pulse wave measuring apparatus according to the present invention, preferably, the air chamber is provided around an entire perimeter of the substrate, for example. In this manner, the entire end surface of the substrate is exposed and faces the air chamber, thereby significantly reducing the stress applied to the substrate. Consequently, the pulse wave can be measured with very high accuracy.

In the pulse wave measuring apparatus according to the present invention, the air chamber is open to atmosphere. When the air chamber is open to atmosphere, air pressure in the air chamber can constantly be maintained to an atmospheric pressure. Accordingly, the stress applied to the substrate is significantly reduced.

The pulse wave measuring apparatus according to the present invention may further include a circuit board processing a signal, and a flexible line transmitting a signal output from the pressure-sensing means to the circuit board. In such a case, the flexible line preferably includes a fixed portion, a connection portion, and a loosened portion. Here, the fixed portion refers to a portion of the flexible line fixed to the protection member, and the connection portion refers to a portion thereof connected to the substrate. The loosened portion is preferably located between the fixed portion and the connection portion. In this manner, by providing the loosened portion in the flexible line, the loosened portion mitigates the stress even when volume fluctuation takes place in the flexible line, thereby significantly reducing the stress applied to the substrate. Consequently, accurate and stable measurement of the pulse wave is allowed.

In the pulse wave measuring apparatus according to the present invention, preferably, the loosened portion is located inside the air chamber, for example. When the loosened portion of the flexible line is located in the air chamber, a larger size of the apparatus due to provision of the loosened portion is avoided, and a compact and high-performance pulse wave measuring apparatus can be provided.

The pulse wave measuring apparatus according to the present invention may further include a circuit board processing a signal, and a flexible line transmitting a signal output from the pressure-sensing means to the circuit board. In such a case, the flexible line preferably includes a fixed portion and a connection portion. Here, the fixed portion refers to a portion of the flexible line fixed to the protection member, and the connection portion refers to a portion thereof connected to the substrate. In addition, preferably, a portion having rigidity different from that of another portion of the flexible line is located between the fixed portion and the connection portion of the flexible line. By providing the portion having rigidity different from that of another portion in the flexible line, the stress is mitigated by this portion even when volume fluctuation takes place in the flexible line, whereby the stress applied to the substrate is significantly reduced. Consequently, accurate and stable measurement of the pulse wave is allowed. Here, in order to provide a portion having rigidity different from that of another portion in the flexible line, a coating of the flexible line is partially removed or its thickness is partially made smaller.

Preferably, the pulse wave measuring apparatus according to the present invention further includes a protection film covering the main surface of the substrate and the air chamber, and attachment means for fastening a peripheral portion of the protection film around an outer circumferential wall of the protection member for attachment. In this manner, by providing the protection film covering the main surface of the substrate, breakage of the pressure-sensing means is prevented. In addition, when the protection film is fastened around the outer circumferential wall of the protection member using the attachment means, detachment of the protection film from the protection member is prevented.

In the pulse wave measuring apparatus according to the present invention, preferably, the protection member has a substantially circular outer shape when viewed from a direction orthogonal to the main surface, of the substrate, and the attachment means is an O ring, for example. When the protection member has a substantially circular outer shape, the protection film can readily be attached to the protection member using the O ring.

In the pulse wave measuring apparatus according to the present invention, preferably, the outer circumferential wall of the protection member has a concave fitting portion fitting to the inner portion of the O ring on an entire circumference, and an outer portion of the O ring projects from the outer circumferential wall of the protection member, for example. In this manner, the concave fitting portion is provided on the outer circumferential wall of the protection member, and the O ring is fitted to the concave fitting portion, thereby ensuring prevention of detachment of the protection film. In addition, as the outer portion of the O ring projects from the outer circumferential wall of the protection member, a sealed system including the pressure-sensing surface can readily be structured by attaching a measurement jig of a cylindrical shape with a bottom to the O ring from a surface side of the substrate so as to achieve intimate contact. Therefore, output characteristics of the pressure-sensing device can readily be measured.

In the pulse wave measuring apparatus according to the present invention, preferably, the protection film and the attachment means are integrally formed, for example. By integrally forming the protection film and the attachment means, the number of parts can be reduced, which leads to facilitated assembly operation and reduction in manufacturing cost.

In the pulse wave measuring apparatus according to the present invention, preferably, the protection film has a collar portion in its peripheral portion, for example. By providing the collar portion in the protection film, the protection film can be attached to the protection member by gripping the collar portion, whereby the assembly operation is facilitated.

In the pulse wave measuring apparatus according to the present invention, preferably, for example, the protection member includes an inner frame body containing the accommodation space and an outer frame body fitted to the inner frame body so as to enclose an outer wall of the inner frame body. Here, the outer frame body preferably has a protection film portion covering the main surface of the pressure-sensing means and the air chamber, and a projected portion provided on an entire circumference of its outer wall. By integrally forming the protection film and the inner frame body, the number of parts can be reduced, which leads to facilitated assembly operation and reduction in manufacturing cost. In addition, by providing the projected portion on the outer circumferential wall of the outer frame body, a sealed system including the pressure-sensing surface can readily be structured by attaching a measurement jig of a cylindrical shape with a bottom to the projected portion from a surface side of the substrate so as to achieve intimate contact. Therefore, the output characteristic of the pressure-sensing device can readily be measured.

The pulse wave measuring apparatus according to the present invention.may further include a circuit board processing a signal, and a flexible line transmitting a signal output from the pressure-sensing means to the circuit board. In such a case, preferably, the protection member includes an inner frame body containing the accommodation space and an outer frame body fitted to the inner frame body so as to enclose an outer wall of the inner frame body, and the flexible line is inserted between the inner frame body and the outer frame body. The flexible line is inserted through the protection member instead of being arranged along the outer wall of the protection member, so that detachment of the flexible line can be prevented.

In the pulse wave measuring apparatus according to the present invention, preferably, the outer frame body has an overhanging portion provided so as to project from an inner surface of the outer frame body and facing, with a distance, a perimeter of an accommodation space forming surface of the inner frame body where the accommodation space is formed, for example. The flexible line inserted between the inner frame body and the outer frame body is preferably protected by the overhanging portion. By providing the overhanging portion protecting the flexible line in the outer frame body, concentration on the flexible line of the pressurization force caused by pressing the pressure-sensing portion against the living body can be avoided, thereby eliminating a possibility of disconnection of the flexible line.

In the pulse wave measuring apparatus according to the present invention, preferably, the protection member is formed with a conductive material. Here, preferably, the protection member is electrically connected to a ground potential. According to such a structure, the pressure-sensing means is less susceptible to static electricity or noise from electric or magnetic field. Therefore, accurate and stable measurement of the pulse wave is allowed.

The pulse wave measuring apparatus according to the present invention may further include a circuit board processing a signal, and a flexible line transmitting a signal output from the pressure-sensing means to the circuit board. Here, preferably, the protection member is electrically connected to the ground potential by means of the flexible line. When the flexible line including a signal line transmitting a signal output from the pressure-sensing means also includes a ground line connecting the protection member to the ground, the number of parts can be reduced, thereby attaining facilitated assembly operation and reduction in manufacturing cost.

In the pulse wave measuring apparatus according to the present invention, preferably, the protection member is formed with a metal material or a ceramic material, for example. In this manner, as heat produced in the pressure-sensing means is effectively dissipated through the protection member, the pulse wave measuring apparatus excellent in safety can be provided.

In the pulse wave measuring apparatus according to the present invention, preferably, the protection member has a plurality of small irregularities on its surface, for example. In this manner, as a surface area of the protection member is increased, heat produced in the pressure-sensing means can effectively be dissipated.

A pulse wave measuring apparatus according to an aspect serves to measure a pulse wave by pressing against a living body a substrate having pressure-sensing means on a main surface. The substrate has a groove around the pressure-sensing means.

In this manner, the groove is provided on the surface of the substrate around the pressure-sensing means so as to implement a thin portion. Then, the stress applied to the end portion of the substrate can be absorbed by the thin portion, and the stress applied to the pressure-sensing means can be reduced. As a result, accurate and stable measurement of the pulse wave is allowed. In addition, as the larger size of the pressure-sensing portion is avoided, a compact and high-performance pulse wave measuring apparatus can be provided.

The pulse wave measuring apparatus according to an aspect may further include a protection member protecting the substrate, a circuit board processing a signal, and a flexible line transmitting a signal output from the pressure-sensing means to the circuit board. In such a case, the flexible line preferably includes a fixed portion, a connection portion, and a loosened portion. Here, the fixed portion refers to a portion of the flexible line fixed to the protection member, and the connection portion refers to a portion thereof connected to the substrate. The loosened portion is preferably located between the fixed portion and the connection portion. In this manner, by providing the loosened portion in the flexible line, the loosened portion mitigates the stress even when volume fluctuation takes place in the flexible line, thereby significantly reducing the stress applied to the substrate. Consequently, the pulse wave can be measured with high accuracy in a stable manner.

The pulse wave measuring apparatus according to an aspect may further include a protection member protecting the substrate, a circuit board processing a signal, and a flexible line transmitting a signal output from the pressure-sensing means to the circuit board. In such a case, the flexible line preferably includes a fixed portion and a connection portion. Here, the fixed portion refers to a portion of the flexible line fixed to the protection member, and the connection portion refers to a portion thereof connected to the substrate. In addition, preferably, a portion having rigidity different from that of another portion of the flexible line is located between the fixed portion and the connection portion of the flexible line. By providing the portion having rigidity different from that of another portion in the flexible line, this portion serves to mitigate the stress even when volume fluctuation takes place in the flexible line, whereby the stress applied to the substrate can significantly be reduced. Consequently, accurate and stable measurement of the pulse wave is allowed. Here, in order to provide a portion having rigidity different from that of another portion in the flexible line, a coating of the flexible line is partially removed or its thickness is partially made smaller.

A pulse wave measuring apparatus according to an aspect includes a substrate having pressure-sensing means on a main surface, a circuit board processing a signal, and a flexible line transmitting a signal output from the pressure-sensing means to the circuit board. The pulse wave measuring apparatus serves to measure a pulse wave by pressing the substrate against a living body. In the pulse wave measuring apparatus according to the third aspect of the present invention, the substrate has a connection electrode portion connected to the flexible line, in a position lower than the main surface.

In this manner, by providing the connection electrode portion in a position lower than the main surface of the substrate, a degree of projection of the flexible line from the main surface of the substrate is suppressed by a distance of lowering. As a result, irregularity on the main surface of the substrate is suppressed, a component of force of the skin tension acting on the pressure-sensing surface is made smaller; and accurate and stable measurement of the pulse wave is allowed.

In the pulse wave Measuring apparatus according to an aspect , preferably, the substrate has a stepped-down portion on its main surface, and the stepped-down portion has the connection electrode portion formed thereon, for example. As to a specific structure for providing the connection electrode portion in a position lower than the main surface of the substrate, it is possible that the stepped-down portion is provided on the main surface of the substrate, and the connection electrode portion is formed on the stepped-down portion, as described above. In this manner, a degree of projection of the flexible line from the main surface of the substrate is suppressed by a distance of lowering. As a result, a component of force of the skin tension acting on the pressure-sensing surface is made smaller, and accurate and stable measurement of the pulse wave is allowed.

In the pulse wave measuring apparatus according to an aspect , preferably, an upper surface of the flexible line located on a side opposite to the connection electrode portion on the stepped-down portion and the main surface of the substrate are located on an identical plane, for example. In this manner, when the upper surface of the flexible line and the main surface of the substrate, that is, the pressure-sensing surface, are located on an identical plane, the skin tension no longer affects the pressure-sensing surface, and accurate and stable measurement of the pulse wave is allowed.

In the pulse wave measuring apparatus according to an aspect , preferably, a spacer member is arranged on the upper surface of the flexible line, and an upper surface of the spacer member located on a side opposite to the flexible line and the main surface of the substrate are located on an identical plane, for example. In this manner, a contact portion with the body surface can be made flat by means of the spacer member.

In the pulse wave measuring apparatus according to an aspect , preferably, the connection electrode portion is formed on a back surface of the substrate, for example. As to another specific structure for providing the connection electrode portion in a position lower than the main surface of the substrate, it is possible that the connection electrode portion is provided on the back surface of the substrate as described above. In order to form the connection electrode portion on the back surface of the substrate, for example, it is possible to provide a through hole in the substrate so as to form a connection contact therein. In this manner, as the flexible line connected to the substrate is no longer located on the main surface of the substrate, a portion of the pressure-sensing portion being in contact with the body surface can be made flat, and accurate and stable measurement of the pulse wave is allowed.

The pulse wave measuring apparatus according to an aspect may further include a protection member protecting the substrate, a circuit board processing a signal, and a flexible line transmitting a signal output from the pressure-sensing means to the circuit board. In such a case, the flexible line preferably includes a fixed portion, a connection portion, and a loosened portion. Here, the fixed portion refers to a portion of the flexible line fixed to the protection member, and the connection portion refers to a portion thereof connected to the substrate. The loosened portion is preferably located between the fixed portion and the connection portion. In this manner, by providing the loosened portion in the flexible line, the loosened portion mitigates the stress even when volume fluctuation takes place in the flexible line, thereby significantly reducing the stress applied on the substrate. Consequently, accurate and stable measurement of the pulse wave is allowed.

The pulse wave measuring apparatus according to an aspect may further include a protection member protecting the substrate, a circuit board processing a signal, and a flexible line transmitting a signal output from the pressure-sensing means to the circuit board. In such a case, the flexible line preferably includes a fixed portion and a connection portion. Here, the fixed portion refers to a portion of the flexible line fixed to the protection member, and the connection portion refers to a portion thereof connected to the substrate. In addition, preferably, a portion having rigidity different from that of another portion of the flexible line is located between the fixed portion and the connection portion of the flexible line. By providing the portion having rigidity different from that of another portion in the flexible line, this portion serves to mitigate the stress even when volume fluctuation takes place in the flexible line, whereby the stress applied to the substrate can significantly be reduced. Consequently, accurate and stable measurement of the pulse wave is allowed. Here, in order to provide a portion having rigidity different from that of another portion in the flexible line, a coating of the flexible line is partially removed or its thickness is partially made smaller.

### Brief Description of the Drawings

Fig. 1 is a schematic perspective view of a pulse wave measuring apparatus in Embodiment 1 of the present invention.
Fig. 2A is a schematic perspective view of a housing portion of the pulse wave measuring apparatus in Embodiment 1 of the present invention.
Fig. 2B is a schematic bottom view of the housing portion of the pulse wave measuring apparatus in Embodiment 1 of the present invention.
Fig. 3A is a schematic diagram illustrating a pressurization mechanism of the pulse wave measuring apparatus in Embodiment 1 of the present invention, before measurement.
Fig. 3B is a schematic diagram illustrating the pressurization mechanism of the pulse wave measuring apparatus in Embodiment 1 of the present invention, during measurement.
Fig. 4 is a schematic cross-sectional view of a structure of a pressure-sensing portion of the pulse wave measuring apparatus in Embodiment 1 of the present invention.
Fig. 5 is an enlarged cross-sectional view of the pressure-sensing portion of the pulse wave measuring apparatus shown in Fig. 4.
Fig. 6 is a schematic cross-sectional view of another portion of the pressure-sensing portion of the pulse wave measuring apparatus shown in Fig. 4.
Fig. 7 is a schematic cross-sectional view of a pressure-sensing portion based on a variation of the pulse wave measuring apparatus in Embodiment 1 of the present invention.
Fig. 8 is a schematic cross-sectional view of a pressure-sensing portion of a pulse wave measuring apparatus in Embodiment 2 of the present invention.
Fig. 9 is a schematic cross-sectional view of a pressure-sensing portion of a pulse wave measuring apparatus in Embodiment 3 (not claimed)
Fig. 10 is an enlarged cross-sectional view of the pressure-sensing portion of the pulse wave measuring apparatus shown in Fig. 9.
Fig. 11 is a schematic perspective view of a semiconductor substrate of the pulse wave measuring apparatus in Embodiment 3.
Fig. 12 is a schematic cross-sectional view of a pressure-sensing portion of a pulse wave measuring apparatus in Embodiment 4 (not claimed).
Fig. 13 is an enlarged cross-sectional view of the pressure-sensing portion of the pulse wave measuring apparatus shown in Fig. 12.
Fig. 14 is a schematic perspective view of a semiconductor substrate of the pulse wave measuring apparatus in Embodiment 4.
Fig. 15 is a schematic cross-sectional view of a pressure-sensing portion of a pulse wave measuring apparatus in Embodiment 5 of the present invention.
Fig. 16 is a schematic cross-sectional view of a pressure-sensing portion of a pulse wave measuring apparatus in Embodiment 6 (not claimed).
Fig. 17 is an enlarged cross-sectional view of the pressure-sensing portion of the pulse wave measuring apparatus shown in Fig. 16.
Fig. 18 is a schematic cross-sectional view of a pressure-sensing portion of a pulse wave measuring apparatus in Embodiment 7 (not claimed).
Fig. 19 is an enlarged cross-sectional view of the pressure-sensing portion of the pulse wave measuring apparatus shown in Fig. 18.
Fig. 20 is a schematic cross-sectional view of a pressure-sensing portion of a pulse wave measuring apparatus in Embodiment 8 of the present invention.
Fig. 21 is a schematic perspective view of a pressure-sensing portion of a pulse wave measuring apparatus in Embodiment 9 of the present invention.
Fig. 22 is a schematic perspective view illustrating a state in which a protection film for the pressure-sensing portion of the pulse wave measuring apparatus shown in Fig. 21 is removed.
Fig. 23 is a schematic cross-sectional view of the pressure-sensing portion of the pulse wave measuring apparatus shown in Fig. 21.
Fig. 24 is an enlarged cross-sectional view of a region XXIV shown in Fig. 23.
Fig. 25 is an exploded perspective view illustrating an assembly structure of the pressure-sensing portion of the pulse wave measuring apparatus shown in Fig. 21.
Fig. 26 is a schematic diagram illustrating a method of measuring an output characteristic of a pressure-sensing device in the pulse wave measuring apparatus shown in Fig. 21.
Fig. 27 is a schematic perspective view illustrating a state in which a protection film is removed, showing a variation of the pulse wave measuring apparatus in Embodiment 9 of the present invention.
Fig. 28 is a schematic cross-sectional view of a pressure-sensing portion of a pulse wave measuring apparatus in Embodiment 10 of the present invention.
Fig. 29 is a schematic diagram illustrating a method of connecting the pressure-sensing portion shown in Fig. 28 to a circuit board.
Fig. 30 is a plan view of a connector portion of a flexible line shown in Fig. 29.
Fig. 31 is a schematic cross-sectional view of a pressure-sensing portion of a pulse wave measuring apparatus in Embodiment 11 of the present invention.
Fig. 32 is a schematic cross-sectional view of a pressure-sensing portion of a pulse wave measuring apparatus in Embodiment 12 of the present invention.
Fig. 33 is a schematic cross-sectional view of a pressure-sensing portion of a conventional pulse wave measuring apparatus.
Fig. 34 is a schematic diagram for pointing out a problem in the conventional pulse wave measuring apparatus.

### Best Modes for Carrying Out the Invention

In the following, the embodiments will be described with reference to the figures.

### (Embodiment 1)

A pulse wave measuring apparatus in Embodiment 1 of the present invention adopts a semiconductor substrate as a substrate and adopts a pressure-sensing device formed on the main surface of the semiconductor substrate as the pressure-sensing means. The pulse wave measuring apparatus adopts a pressure-sensing device utilizing a diaphragm, for example. The pulse wave measuring apparatus in the present embodiment is a pressurization-type pulse wave measuring apparatus for measuring the pulse wave by pressing the main surface of the semiconductor substrate against the body surface.

### (Overall Structure)

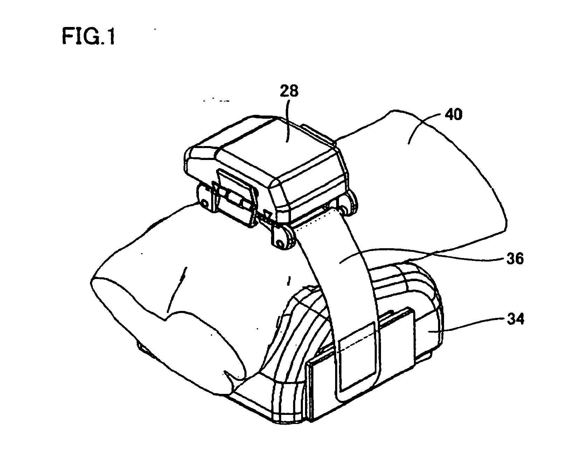

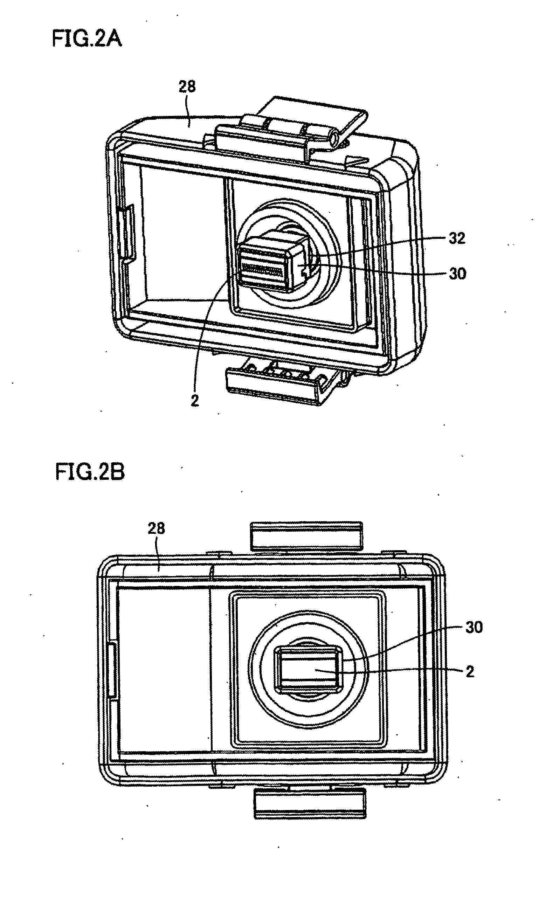

Referring first to Figs. 1, 2A and 2B, an overall structure of the pulse wave measuring apparatus in Embodiment 1 according to the present invention will be described. Fig. 1 is a schematic perspective view showing the overall structure of the pulse wave measuring apparatus in Embodiment 1 of the present invention. Fig. 2A is a schematic perspective view of a structure of a housing portion of the pulse wave measuring apparatus in the present embodiment, and Fig. 2B is a bottom view of the housing portion.

As shown in Fig: 1, the pulse wave measuring apparatus in the present embodiment includes a securing base 34, a housing portion 28, and a band 36. Securing base 34 serves to fix a measurement site of a living body 40 of the subject. In the pulse wave measuring apparatus shown in Fig. 1, a wrist of the subject is adopted as the measurement site subjected to measurement of the pulse wave. Therefore, securing base 34 is shaped so as to be able to fix the wrist.

Band 36 is attached to a prescribed position of securing base 34. In addition, housing portion 28 is attached to band 36. Housing portion 28 has a pressure-sensing portion 30 (see Figs. 2A and 2B) on its lower surface as will be described later. Therefore, by winding band 36 around the wrist placed on securing base 34, pressure-sensing portion 30 of housing portion 28 is located on the measurement site of the subject.

As shown in Figs. 2A and 2B, pressure-sensing portion 30 including a pressure-sensing surface 2 is arranged on the lower surface side of housing portion 28. An air bag 32 for pressing pressure-sensing portion 30 against the living body is attached to pressure-sensing portion 30. Here, pressure-sensing portion 30 is supported in a manner movable in an up-down direction.

### (Pressurization Mechanism)

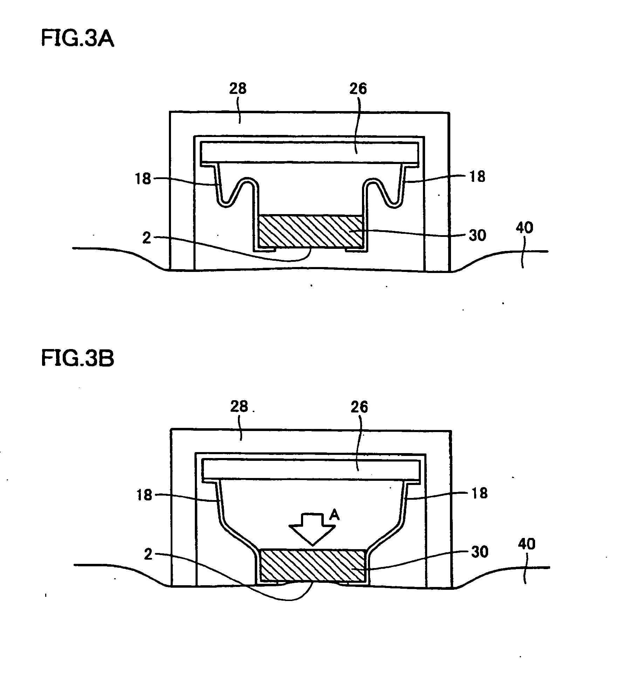

Referring now to Figs. 3A and 3B, a pressurization mechanism in the pulse wave measuring apparatus in the present embodiment will be described. Figs. 3A and 3B are schematic diagrams showing the pressurization mechanism of the pulse wave measuring apparatus in the present embodiment. Fig. 3A is a schematic diagram illustrating a state before measurement, and Fig. 3B is a schematic diagram illustrating a state during measurement.

As shown in Figs. 3A and 3B, a circuit board 26 is arranged inside housing portion 28 of the pulse wave measuring apparatus. A processing circuit processing a signal output from the pressure-sensing device is formed on circuit board 26. In transmission of the signal output from the pressure-sensing device, a flexible line 18 is used. Flexible line 18 has one end electrically connected to pressure-sensing portion 30 having the pressure-sensing device, and the other end electrically connected to circuit board 26.

As shown in Fig. 3A, before measurement, pressure-sensing portion 30 is arranged in a position distant from body surface 40. Here, flexible line 18 has an excessive portion, and is loosened between pressure-sensing portion 30 and circuit board 26. During measurement, as the not-shown air bag expands, pressure-sensing portion 30 is moved in the direction shown with arrow A as shown in Fig. 3B, and pressure-sensing surface 2 of the pressure-sensing portion 30 is pressed against body surface 40. In such a state, the pulse wave produced in the artery located directly under the skin, that is, body surface 40, can be detected using the pressure-sensing device.

### (Structure of Pressure-Sensing Portion)

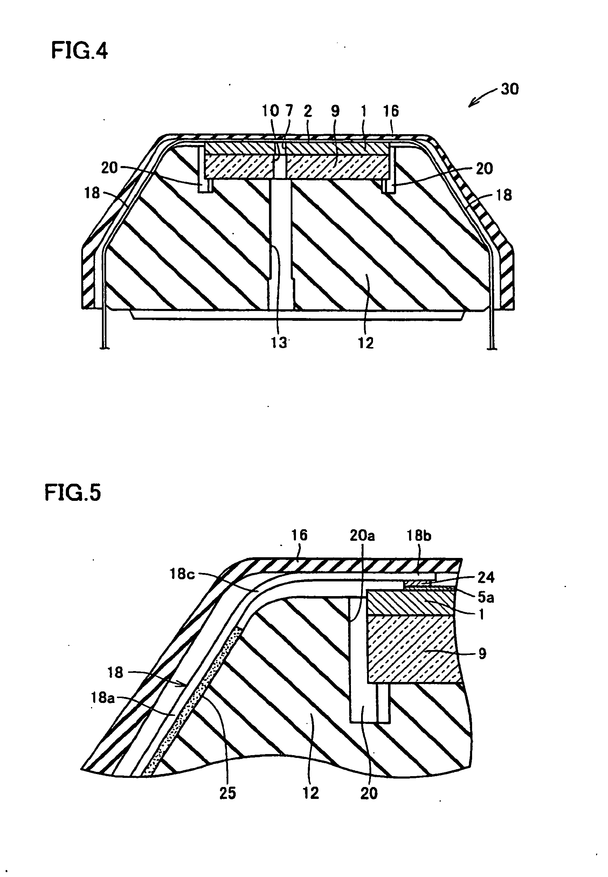

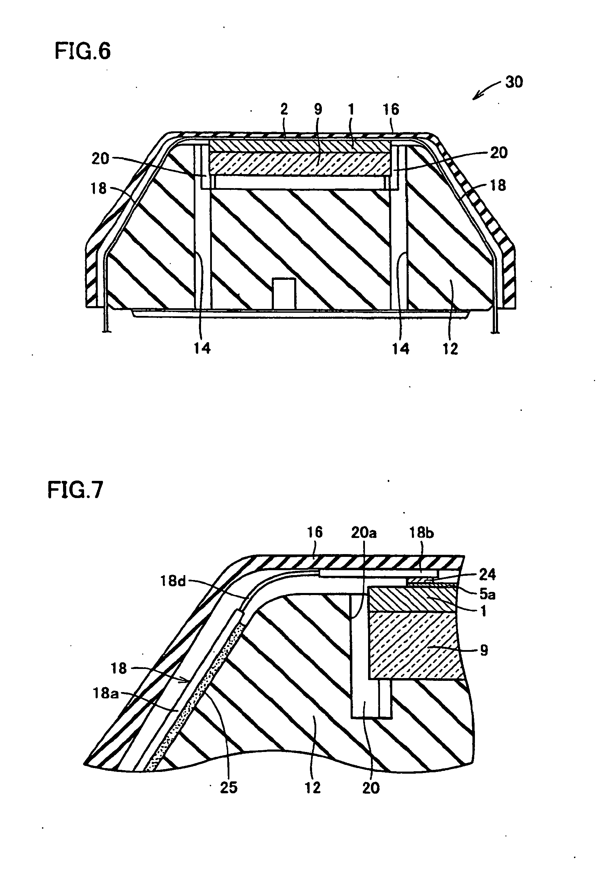

A structure of the pressure-sensing portion of the pulse wave measuring apparatus in the present embodiment will now be described in detail. Fig. 4 is a schematic cross-sectional view of the pulse wave measuring apparatus in the present embodiment, and Fig. 5 is an enlarged cross-sectional view of the pressure-sensing portion shown in Fig. 4. Fig. 6 is a schematic cross-sectional view of another portion of the pressure-sensing portion of the pulse wave measuring apparatus shown in Fig. 4.

As shown in Figs. 4 and 5, pressure-sensing portion 30 mainly includes semiconductor substrate 1 having the pressure-sensing device formed on the main surface, support member 9 supporting a back surface of semiconductor substrate 1, protection member 12 holding support member 9 and protecting semiconductor substrate 1, flexible line 18 electrically connected to semiconductor substrate 1, and protection film 16 attached to a contact portion with the body surface of pressure-sensing portion 30.

Protection member 12 is fabricated with a resin member having a substantially trapezoidal shape, and has an accommodation space accommodating semiconductor substrate 1 in its surface. In the present embodiment, the accommodation space is formed by a concave portion formed in the surface of protection member 12. On the bottom surface of the concave portion, support member 9 is disposed. Support member 9 is a plate-shaped member attaining a function as an insulating member, and a glass plate or an anodized aluminum plate is used as support member 9, for example. Semiconductor substrate 1 is adhered to the upper surface of support member 9. Adhesion is carried out by anodic bonding, for example.

As shown in Fig. 4, protection member 12 has a communication hole 13 for introducing atmosphere formed. Communication hole 13 reaches the lower surface of support member 9 arranged in the concave portion of protection member 12. Support member 9 has a communication hole 10 formed. Communication hole 10 communicates to communication hole 13 provided in projection member 12 described above, and reaches the lower surface of semiconductor substrate 1 arranged on support member 9. A small hole 7 is provided in a prescribed region of the lower surface of semiconductor substrate 1, and communicates to communication hole 10 provided in support member 9 described above. A diaphragm which is a portion of the pressure-sensing device is formed in an upper portion of small hole 7. In this manner, communication holes 13, 10 and small hole 7 are provided so as to introduce atmosphere therethrough, so that the lower surface of the diaphragm is maintained to an atmospheric pressure.

As shown in Fig. 5, flexible line 18 has one end brazed to a connection electrode portion 5a provided on the main surface of semiconductor substrate 1 by a brazing material 24, and has the other end electrically connected a not-shown circuit board. The flexible line is implemented by coating and supporting a plurality of foil-like wires with a flexible sheet, and generally referred to as a flexible flat cable. Flexible line 18 is drawn out from the end portion of semiconductor substrate 1 toward the side surface of protection member 12, and fixed to protection member 12 by an adhesive 25.

Here, flexible line 18 includes a fixed portion 18a fixed to protection member 12 by adhesive 25, a connection portion 18b connected to semiconductor substrate 1 by brazing material 24, and a loosened portion 18c located between fixed portion 18a and connection portion 18b and arranged in a slightly loosened manner. By providing loosened portion 18c, loosened portion 18c mitigates the stress even when volume fluctuation occurs in flexible line 18, and direct application of the stress to semiconductor substrate 1 is avoided.

### (Structure of Air Chamber)

As shown in Fig. 5, a wall surface 20a of the concave portion forming the accommodation space of protection member 12 is arranged such that an air chamber 20 is interposed between the wall surface and the end surface of semiconductor substrate 1. In other words, wall surface 20a of protection member 12 and the end surface of semiconductor substrate 1 are arranged spaced apart from each other, so as to form air chamber 20. In the present embodiment, air chamber 20 is provided around the entire perimeter of semiconductor substrate 1.

As shown in Fig. 6, air chamber 20 is open to the atmosphere through a communication hole 14 provided in protection member 12. In this manner, the air in air chamber 20 is constantly maintained to the atmospheric pressure. In the pulse wave measuring apparatus in the present embodiment, the end surface of support member 9 is also structured to face air chamber 20.

### (Function and Effect)

As described above, in the pulse wave measuring apparatus in the present embodiment, the end surface of the semiconductor substrate having the pressure-sensing device formed on the main surface is surrounded by the air chamber. Accordingly, as compared with the pulse wave measuring apparatus in which another member is arranged on the end surface of the semiconductor substrate, the stress is not applied to the semiconductor substrate even when variation in the ambient temperature or heat transfer from the body surface takes place. Therefore, accurate and stable measurement of the pulse wave is allowed.

In addition, by covering the end surface of the semiconductor substrate with the air chamber, deformation of the semiconductor substrate in the lateral direction caused by pressurization of the semiconductor substrate against the body surface is no longer suppressed. Accordingly, application of the stress from the end surface of the semiconductor substrate to the substrate is avoided. Consequently, accurate and stable measurement of the pulse wave is allowed.

Moreover, as the semiconductor substrate is disposed in the concave portion serving as the accommodation space of the protection member, protection of the semiconductor substrate by the protection member is ensured. Therefore, a compact and high-performance pulse wave measuring apparatus can be provided.

Fig. 7 is a schematic cross-sectional view of the pressure-sensing portion based on a variation of the pulse wave measuring apparatus in the present embodiment. As shown in Fig. 6, the structure in which flexible line 18 includes loosened portion 18c in order to avoid application of the stress from flexible line 18 to semiconductor substrate 1 has been described by way of example. As shown in Fig. 7, however, a portion 18d having rigidity different from that of another portion may be formed between fixed portion 18a and connection portion 18b of flexible line 18. Examples of a method of forming portion 18d having rigidity different from that of another portion include a method of partially removing a coating of flexible line 18 so as to expose the line, and a method of partially reducing a thickness of the coating of flexible line 18.

### (Embodiment 2)

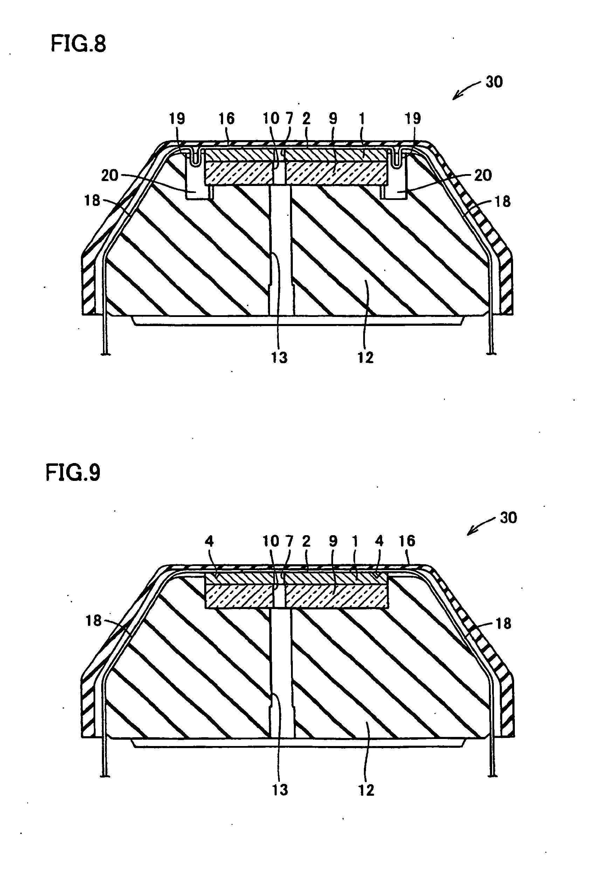

A structure of a pressure-sensing portion of a pulse wave measuring apparatus in Embodiment 2 of the present invention will now be described in detail. Fig. 8 is a schematic cross-sectional view of the pressure-sensing portion of the pulse wave measuring apparatus in Embodiment 2 of the present invention. The pulse wave measuring apparatus in the present embodiment adopts a pressure-sensing device formed on the main surface of the semiconductor substrate as the pressure-sensing means, as in Embodiment 1 described above. It is noted that the same reference characters are given to components the same or corresponding to those in Embodiment 1 described above, and description thereof will not be repeated.

### (Structure of Pressure-Sensing Portion)

As shown in Fig. 8, the pulse wave measuring apparatus in the present embodiment has air chamber 20 formed so as to face the end surface of semiconductor substrate 1 as in Embodiment 1 described above. In the pulse wave measuring apparatus in the present embodiment, flexible line 18 includes a loosened portion 19 formed by bending the line in a degree larger than loosened portion 18c in Embodiment 1 described above, between fixed portion 18a and connection potion 18b. Loosened portion 19 is arranged in air chamber 20.

### (Function and Effect)

As described above, the loosened portion provided in the flexible line is arranged in the air chamber, so that a larger loosened portion can be provided. If a larger loosened portion is provided, correspondingly, the stress applied to the semiconductor substrate can further be reduced. Therefore, more accurate and stable measurement of the pulse wave is allowed. In addition, by arranging the loosened portion in the air chamber, a larger size of the apparatus due to provision of the loosened portion is avoided, whereby a compact and high-performance pulse wave measuring apparatus can be provided.

### (Embodiment 3)

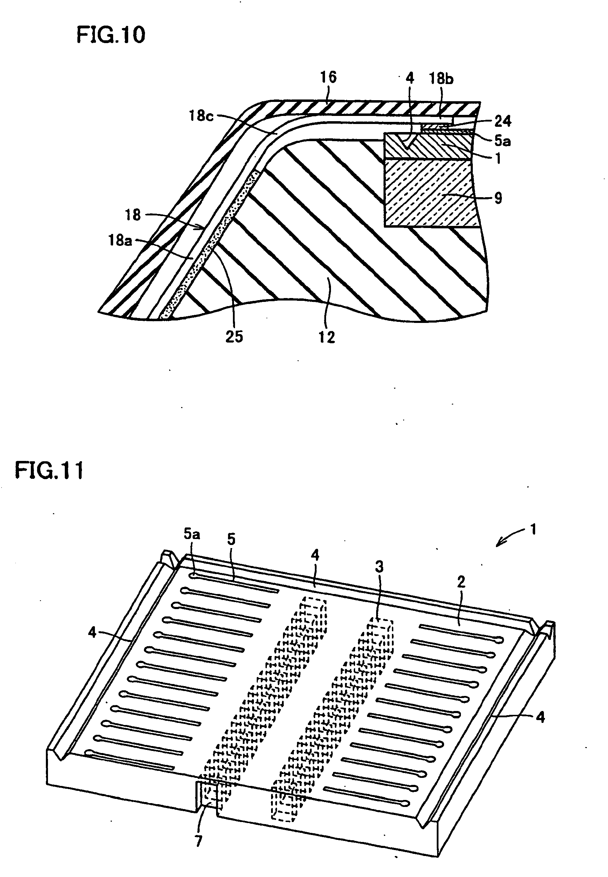

A structure of a pulse wave measuring apparatus in Embodiment 3 will now be described in detail. Fig. 9 is a schematic cross-sectional view of a pressure-sensing portion of the pulse wave measuring apparatus in Embodiment 3 . Fig. 10 is an enlarged cross-sectional view of the pressure-sensing portion shown in Fig. 9. Fig. 11 is a schematic perspective view of a structure of a semiconductor substrate of the pulse wave measuring apparatus shown in Fig. 9. The pulse wave measuring apparatus in the present embodiment adopts a pressure-sensing device formed on the main surface of the semiconductor substrate as the pressure-sensing means, as in Embodiment 1 described above. It is noted that the same reference characters are given to components the same or corresponding to those in Embodiment 1 described above, and description thereof will not be repeated.

### (Structure of Pressure-Sensing Portion)

As shown in Figs. 9 and 10, pressure-sensing portion 30 of the pulse wave measuring apparatus in the present embodiment, likewise in Embodiment 1 described above, mainly includes semiconductor substrate 1 having the pressure-sensing device formed on the main surface, support member 9 supporting a back surface of semiconductor substrate 1, protection member 12 holding support member 9 and protecting semiconductor substrate 1, flexible line 18 electrically connected to semiconductor substrate 1, and protection film 16 attached to a contact portion with the body surface of pressure-sensing portion 30.

In pressure-sensing portion 30 of the pulse wave measuring apparatus in the present embodiment, protection member 12 is arranged so as to be in contact with the end surface of semiconductor substrate 1. Accordingly, semiconductor substrate 1 is protected by protection member 12.

### (Structure of Semiconductor Substrate)

As shown in Fig. 11, semiconductor substrate 1 has a plurality of pressure-sensing devices 3 on the main surface. The plurality of pressure-sensing devices 3 are arranged in the vicinity of a central portion of semiconductor substrate 1. In a prescribed region of the main surface of semiconductor substrate 1, an interconnection 5 formed of a conductor film for transmitting a signal output from pressure-sensing device 3 to the outside is formed. Interconnection 5 is connected to connection electrode portion 5a also formed of a conductor film. One end of flexible line 18 is brazed to connection electrode portion 5a using brazing material 24 (see Fig. 10).

A groove 4 is provided on the main surface of semiconductor substrate 1 so as to surround pressure-sensing device 3. Groove 4 implements a thin portion along the perimeter of semiconductor substrate 1. In semiconductor substrate 1 shown in Fig. 11, groove 4 is provided along three sides of semiconductor substrate 1, and the thin portions extend in three directions of pressure-sensing device 3.

### (Function and Effect)

As described above, in the pulse wave measuring apparatus in the present embodiment, the groove is formed so as to surround the pressure-sensing device on the main surface of the semiconductor substrate, and the groove implements the thin portion. Therefore, even when volume fluctuation of the protection member takes place due to variation in the ambient temperature or heat transfer from the body surface, the stress applied from the protection member to the semiconductor substrate is mitigated by the thin portion, and accurate and stable measurement of the pulse wave is allowed.

In addition, by providing the thin portion on the main surface of the semiconductor substrate, deformation of the semiconductor substrate in the lateral direction caused by pressurization of the semiconductor substrate against the body surface is less likely to be restricted, and the stress applied from the end surface of the semiconductor substrate to the substrate is mitigated by the thin portion. Consequently, accurate and stable measurement of the pulse wave is allowed.

Moreover, in the present embodiment, the stress is mitigated by such a simple structure as the groove in the semiconductor substrate. Therefore, a compact and high-performance pulse wave measuring apparatus can be provided.

As shown in Fig. 10, in the present embodiment, similarly to Embodiment 1 described above, flexible line 18 includes loosened portion 18c in order to avoid application of the stress from flexible line 18 to semiconductor substrate 1. Alternatively, a portion having rigidity different from that of another portion may be formed between fixed portion 18a and connection portion 18b of flexible line 18.

### (Embodiment 4)

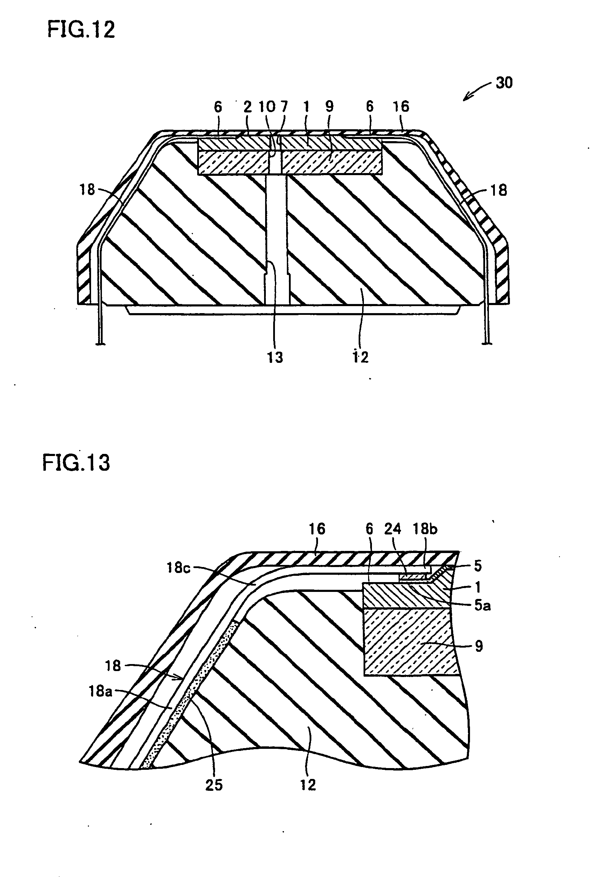

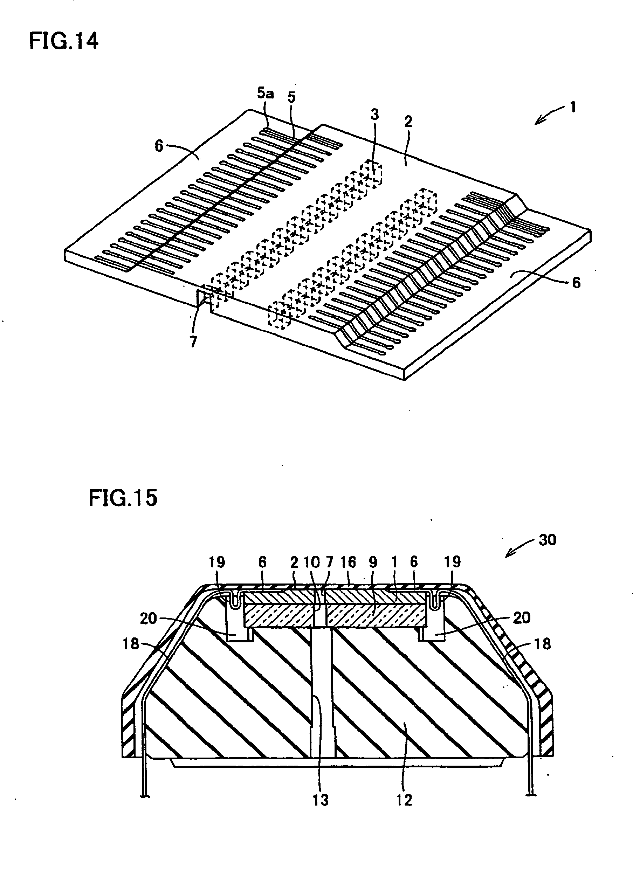

A structure of a pulse wave measuring apparatus in Embodiment 4 will now be described in detail. Fig. 12 is a schematic cross-sectional view of a pressure-sensing portion of the pulse wave measuring apparatus in Embodiment 4 . Fig. 13 is an enlarged cross-sectional view of the pressure-sensing portion shown in Fig. 12. Fig. 14 is a schematic perspective view of a structure of the semiconductor substrate of the pulse wave measuring apparatus shown in Fig. 12. The pulse wave measuring apparatus in the present embodiment adopts a pressure-sensing device formed on the main surface of the semiconductor substrate as the pressure-sensing means, as in Embodiment 1 described above. It is noted that the same reference characters are given to components the same or corresponding to those in Embodiment 1 described above, and description thereof will not be repeated

### (Structure of Pressure-Sensing Portion)

As shown in Figs. 12 and 13, pressure-sensing portion 30 of the pulse wave measuring apparatus in the present embodiment, likewise in Embodiment 1 described above, mainly includes semiconductor substrate 1 having the pressure-sensing device formed on the main surface, support member 9 supporting a back surface of semiconductor substrate 1, protection member 12 holding support member 9 and protecting semiconductor substrate 1, flexible line 18 electrically connected to semiconductor substrate 1, and protection film 16 attached to a contact portion with the body surface of pressure-sensing portion 30.

In pressure-sensing portion 30 of the pulse wave measuring apparatus in the present embodiment, protection member 12 is arranged so as to be in contact with the end surface of semiconductor substrate 1. Accordingly, semiconductor substrate 1 is protected by protection member 12.

### (Structure of Semiconductor Substrate)

As shown in Fig. 14, semiconductor substrate 1 has a plurality of pressure-sensing devices 3 on the main surface. The plurality of pressure-sensing devices 3 are arranged in the vicinity of the central portion of semiconductor substrate 1. In a prescribed region of the main surface of semiconductor substrate 1, interconnection 5 formed of a conductor film for transmitting a signal output from pressure-sensing device 3 to the outside is formed.

In a prescribed region of semiconductor substrate 1, a stepped-down portion 6 is provided. Stepped-down portion 6 includes a stepped-down surface lower than pressure-sensing surface 2 serving as the main surface of semiconductor substrate 1, and connection electrode portion 5a formed of a conductor film is formed on the stepped-down surface. Connection electrode portion 5a is connected to interconnection 5 describe above. One end of flexible line 18 is brazed to connection electrode portion 5a using brazing material 24 (see Fig. 13). In semiconductor substrate 1 shown in Fig. 13, stepped-down portions 6 are provided on opposing sides of semiconductor substrate 1.

### (Structure in the Vicinity of Connection Electrode Portion)

As shown in Fig. 13, in pressure-sensing portion 30 of the pulse wave measuring apparatus in the present embodiment, semiconductor substrate 1 including stepped-down portion 6 described above is arranged on support member 9. In stepped-down portion 6, flexible line 18 is located on connection electrode portion 5a. Here, the upper surface of flexible line 18 located on a side opposite to connection electrode portion 5a and the main surface of semiconductor substrate 1 are located on a substantially identical plane. In other words, a portion being in contact with the lower surface of protection film 16 has a substantially flat shape.

### (Function and Effect)

As described above, in the pulse wave measuring apparatus in the present embodiment, the surface of the pressure-sensing portion pressed against the body surface has a substantially flat shape, and application of a component of force of the skin tension to the semiconductor substrate is avoided. Therefore, accurate and stable measurement of the pulse wave is allowed.

As shown in Fig. 13, in the present embodiment, similarly to Embodiment 1 described above, flexible line 18 includes loosened portion 18c in order to avoid application of the stress from flexible line 18 to semiconductor substrate 1. Alternatively, a portion having rigidity different from that of another portion may be formed between fixed portion 18a and connection portion 18b of flexible line 18.

### (Embodiment 5)

A structure of a pressure-sensing portion of the pulse wave measuring apparatus in Embodiment 5 will now be described in detail. Fig. 15 is a schematic cross-sectional view of the pressure-sensing portion of the pulse wave measuring apparatus in Embodiment 5 of the present invention. The pulse wave measuring apparatus in the present embodiment adopts a pressure-sensing device formed on the main surface of the semiconductor substrate as the pressure-sensing means, as in Embodiment 4 described above. It is noted that the same reference characters are given to components the same or corresponding to those in Embodiment 4 described above, and description thereof Will not be repeated.

### (Structure of Pressure-Sensing Portion)

As shown in Fig. 15, the pulse wave measuring apparatus in the present embodiment has a structure implemented by combining Embodiments 2 and 4 described above. Specifically, semiconductor substrate 1 has stepped-down portion 6 on the main surface, and connection electrode portion 5a is located on stepped-down portion 6. Flexible line 18 is located on connection electrode portion 5a, and the upper surface of flexible line 18 and the main surface of semiconductor substrate 1 are located on a substantially identical plane. In addition, air chamber 20 is formed so as to face the end surface of semiconductor substrate 1. Loosened portion 19 formed by bending flexible line 18 in a larger degree is arranged in air chamber 20.

### (Function and Effect)

As described above, in the pulse wave measuring apparatus in the present embodiment, the surface of the pressure-sensing portion pressed against the body surface has a substantially flat shape by providing the stepped-down portion in the semiconductor substrate. Accordingly, the pressure-sensing portion is less susceptible to the component of force of the skin tension. In addition, as the end surface of the semiconductor substrate is surrounded by the air chamber, the stress applied to the end surface of the semiconductor substrate is significantly reduced as compared with the pulse wave measuring apparatus in which another member is arranged on the end surface of the semiconductor substrate. Moreover, as the loosened portion is provided in the flexible line, application of the stress from the flexible line to the semiconductor substrate can be avoided. In this manner, a variety of forces applied to the semiconductor substrate are eliminated, and therefore, highly accurate and stable measurement of the pulse wave is allowed.

### (Embodiment 6)

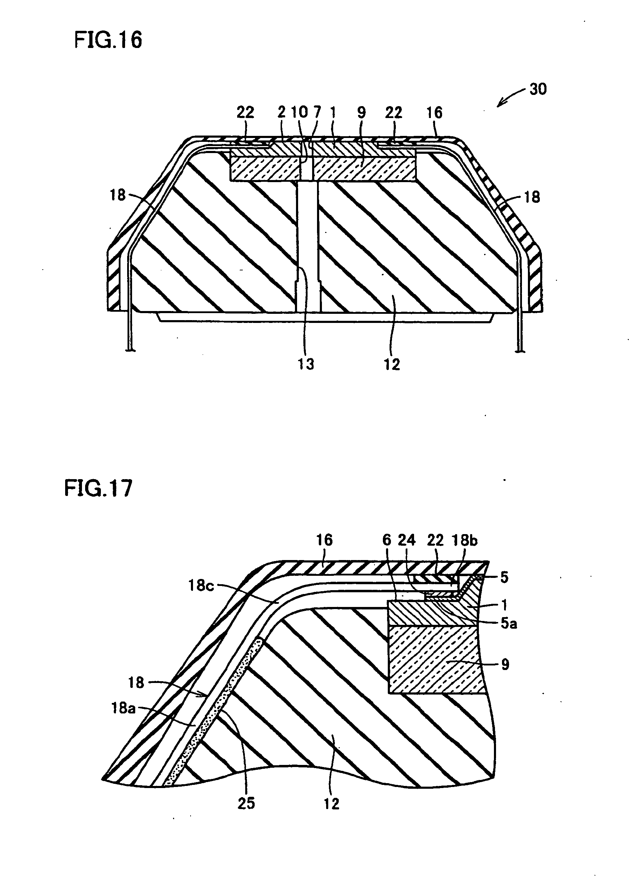

A structure of the pulse wave measuring apparatus in Embodiment 6 will now be described in detail. Fig. 16 is a schematic cross-sectional view of a pressure-sensing portion of the pulse wave measuring apparatus in Embodiment 6. Fig. 17 is an enlarged cross-sectional view of the pressure-sensing portion shown in Fig. 16. The pulse wave measuring apparatus in the present embodiment adopts a pressure-sensing device formed on the main surface of the semiconductor substrate as the pressure-sensing means, as in Embodiment 4 described above. It is noted that the same reference characters are given to components the same or corresponding to those in Embodiment 4 described above, and description thereof will not be repeated.

### (Structure of Pressure-Sensing Portion)

As shown in Figs. 16 and 17, in the pulse wave measuring apparatus in the present embodiment, a height of the step provided in semiconductor substrate 1 is larger than that in the pulse wave measuring apparatus in Embodiment 4 described above. Flexible line 18 is arranged on connection electrode portion 5a on stepped-down portion 6. In addition, a spacer member 22 is arranged on flexible line 18. Here, the upper surface of spacer member 22 located on a side opposite to flexible line 18 and the main surface of semiconductor substrate 1 are located on a substantially identical plane. In other words, a portion being in contact with the lower surface of protection film 16 has a substantially flat shape.

### (Function and Effect)

As described above, in the pulse wave measuring apparatus in the present embodiment, the surface of the pressure-sensing portion pressed against the body surface has a substantially flat shape by employing the spacer member, and application of the component of force of the skin tension to the semiconductor substrate can be avoided. Therefore, accurate and stable measurement of the pulse wave is allowed.

### (Embodiment 7)

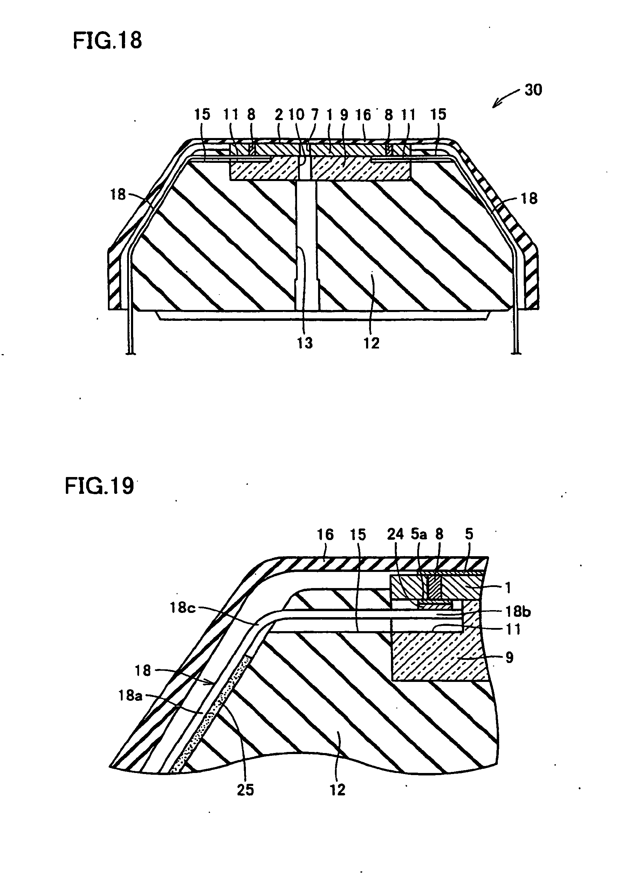

A structure of the pulse wave measuring apparatus in Embodiment 7 will now be described in detail. Fig. 18 is a schematic cross-sectional view of a pressure-sensing portion of the pulse wave measuring apparatus in Embodiment 7. Fig. 19 is an enlarged cross-sectional view of the pressure-sensing portion shown in Fig. 18. The pulse wave measuring apparatus in the present embodiment adopts a pressure-sensing device formed on the main surface of the semiconductor substrate as the pressure-sensing means, as in Embodiment 1 described above. It is noted that the same reference characters are given to components the same or corresponding to those in Embodiment 1 described above, and description thereof will not be repeated.

### (Structure of Pressure-Sensing Portion)

As shown in Figs. 18 and 19, pressure-sensing portion 30 of the pulse wave measuring apparatus in the present embodiment, likewise in Embodiment 1 described above, mainly includes semiconductor substrate 1 having the pressure-sensing device formed on the main surface, support member 9 supporting a back surface of semiconductor substrate 1, protection member 12 holding support member 9 and protecting semiconductor substrate 1, flexible line 18 electrically connected to semiconductor substrate 1, and protection film 16 attached to a contact portion with the body surface of pressure-sensing portion 30.

In pressure-sensing portion 30 of the pulse wave measuring apparatus in the present embodiment, protection member 12 is arranged so as to be in contact with the end surface of semiconductor substrate 1. Accordingly, semiconductor substrate 1 is protected by protection member 12.

### (Structure of Semiconductor Substrate)

As shown in Figs. 18 and 19, in a prescribed region of the main surface of semiconductor substrate 1, interconnection 5 formed of a conductor film for transmitting a signal output from pressure-sensing device 3 to the outside is formed. Interconnection 5 is connected to connection electrode portion 5a provided on the back surface of semiconductor substrate 1 through a connection contact 8 provided in semiconductor substrate 1. In other words, connection electrode portion 5a is formed in a position lower than the main surface of semiconductor substrate 1. Connection contact 8 is implemented as a plug formed by filling a through hole provided in semiconductor substrate 1 with a conductive member.

Support member 9 has a notch 11 in a position corresponding to connection electrode portion 5a provided on the back surface of semiconductor substrate 1. As such, one end of flexible line 18 can be connected to connection electrode portion 5a, and flexible line 18 is brazed to connection electrode portion 5a with brazing material 24 on the back surface of semiconductor substrate 1. Here, flexible line 18 is drawn out of the side surface of protection member 12 through an insertion hole 15 provided in protection member 12.

### (Function and Effect)

As described above, in the pulse wave measuring apparatus of the present embodiment, by providing the connection electrode portion on the back surface of the semiconductor substrate, the flexible line is not located on the main surface of the semiconductor substrate. Accordingly, the surface of the pressure-sensing portion pressed against the body surface has a substantially flat shape, and application of the component of force of the skin tension to the semiconductor substrate can be avoided. Therefore, accurate and stable measurement of the pulse wave is allowed.

As shown in Fig. 19, in the present embodiment, similarly to Embodiment 1 described above, flexible line 18 includes loosened portion 18c in order to avoid application of the stress from flexible line 18 to semiconductor substrate 1. Alternatively, a portion having rigidity different from that of another portion may be formed between fixed portion 18a and connection portion 18b of flexible line 18.

### (Embodiment 8)

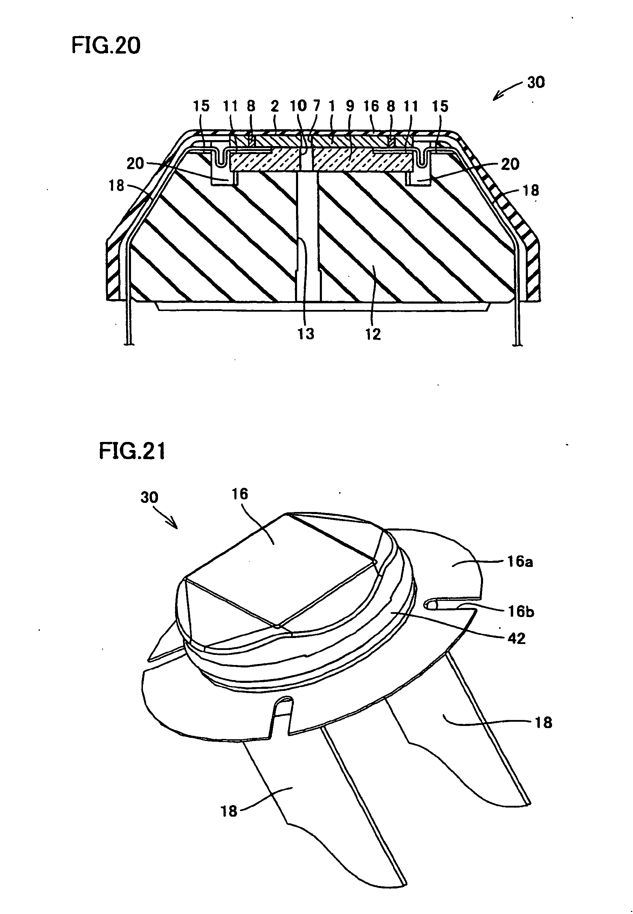

A structure of a pressure-sensing portion of the pulse wave measuring apparatus in Embodiment 8 of the present invention will now be described in detail. Fig. 20 is a schematic cross-sectional view of the pressure-sensing portion of the pulse wave measuring apparatus in Embodiment 8 of the present invention. The pulse wave measuring apparatus in the present embodiment adopts a pressure-sensing device formed on the main surface of the semiconductor substrate as the pressure-sensing means, as in Embodiment 7 described above. It is noted that the same reference characters are given to components the same or corresponding to those in Embodiment 7 described above, and description thereof will not be repeated.

### (Structure of Pressure-Sensing Portion)

As shown in Fig. 20, the pulse wave measuring apparatus in the present embodiment has a structure implemented by combining Embodiments 2 and 7 described above. Specifically, semiconductor substrate 1 has connection contact 8 formed by filling the through hole with the conductive member, and has connection electrode portion 5a on the back surface. Flexible line 18 is connected to connection electrode portion 5a. In addition, air chamber 20 is formed so as to face the end surface of semiconductor substrate 1. Loosened portion 19 formed by bending flexible line 18 in a larger degree is arranged in air chamber 20.

### (Function and Effect)

As described above, in the pulse wave measuring apparatus of the present embodiment, by providing the connection electrode portion on the back surface of the semiconductor substrate, the flexible line is not located on the main surface of the semiconductor substrate. Accordingly, the surface of the pressure-sensing portion pressed against the body surface has a substantially flat shape, and application of the component of force of the skin tension to the semiconductor substrate can be avoided. As the end surface of the semiconductor substrate is surrounded by the air chamber, the stress applied to the end surface of the semiconductor substrate is significantly reduced as compared with the pulse wave measuring apparatus in which another member is arranged on the end surface of the semiconductor substrate. Moreover, as the loosened portion is provided in the flexible line, application of the stress from the flexible line to the semiconductor substrate can also be avoided. In this manner, a variety of forces applied to the semiconductor substrate are eliminated, and therefore, highly accurate and stable measurement of the pulse wave is allowed.

### (Further Problems of Pulse Wave Measuring Apparatus Shown in Embodiments 1 to 8)

In the pulse wave measuring apparatus having the structure shown in Embodiments 1 to 8 described above, a variety of forces applied to the end portion of the semiconductor substrate can be eliminated, and drastic improvement in measurement accuracy can effectively be achieved as compared with the conventional pulse wave measuring apparatus. On the other hand, further improvement is still required in the following points.

First, when the pulse wave measuring apparatus is repeatedly used, the protection film attached to the protection member so as to cover the pressure-sensing surface may detach. This is because the flexible line is bent by repeated elevation and lowering of the pressure-sensing portion and it comes off from a sidewall of the protection member due to the pressurization force from the protection film.

Secondly, accurate measurement of output characteristics of the pressure-sensing device is difficult in an inspection process before product shipment carried out in order to grasp variation in the output characteristics of the pressure-sensing device. As described above, when the pressure-sensing device formed on the main surface of the semiconductor substrate is used as the pressure-sensing means, output characteristics of individual sensor chips may be different from one another due to fluctuation in manufacturing conditions or the like. Accordingly, for highly accurate measurement of the pulse wave, it is necessary to grasp the output characteristics of the individual sensor chips as well as to correct a measurement value as required. Here, measurement of the output characteristics of the pressure-sensing device is carried out, for example, by arranging a pressure-sensing portion in the sealed system, increasing a pressure in the system so as to apply a prescribed pressure to the pressure-sensing surface, and measuring an output. In the pulse wave measuring apparatus having the structure shown in Embodiments 1 to 8 described above, however, it is difficult to implement the sealed system due to a structural problem, and to accurately measure the output characteristics.

Thirdly, there is a possibility of disconnection of the flexible line at the end portion of the semiconductor substrate. In the pulse wave measuring apparatus shown in Embodiments 7 and 8 described above, the connection electrode portion connected to the flexible line is provided on the back surface of the semiconductor substrate. Accordingly, application of the pressurization force produced by pressing the pressure-sensing portion against the living body to the flexible line is avoided, and consequently, disconnection of the flexible line is unlikely. In the pulse wave measuring apparatus shown in Embodiments 1 to 6 described above, however, the connection electrode portion connected to the flexible line is located on the main surface side of the semiconductor substrate. Then, the flexible line is provided on the main surface of the semiconductor substrate, and accordingly, the pressurization force produced by pressing the pressure-sensing portion against the living body concentrates on a portion of the flexible line located at the end portion of the semiconductor substrate, resulting in disconnection. In particular, when the air chamber is provided around the semiconductor substrate as in the pulse wave measuring apparatus shown in Embodiments 1, 2 and 5, the stress may concentrate not only on the portion of the flexible line located at the end portion of the semiconductor substrate but also on a portion of the flexible line located at the end portion of the protection member, due to introduction of the skin in the air chamber at the time of pressurization. In such a case, disconnection of the flexible line is further likely.

Fourthly, there is a problem of susceptibility to static electricity or noise from electric or magnetic field. In the pulse wave measuring apparatus shown in Embodiments 1 to 8 described above, the main surface of the semiconductor substrate is merely covered with a thin protection film. Therefore, such a pulse wave measuring apparatus is susceptible to static electricity or noise from electric or magnetic field, and accurate measurement of the pulse wave cannot be carried out under such an external influence.

Fifthly, there remains a problem of safety. During measurement, a current flows in the pressure-sensing device and the temperature of the semiconductor substrate is slightly increased. Though such temperature increase is less likely to cause a problem under a room temperature, a possibility of cold burn cannot be denied if measurement is performed under a high temperature.

In the following, a pulse wave measuring apparatus overcoming these various problems by further improving the pulse wave measuring apparatuses in Embodiments 1 to 8 described above will be described in detail with reference to the drawings.

### (Embodiment 9)

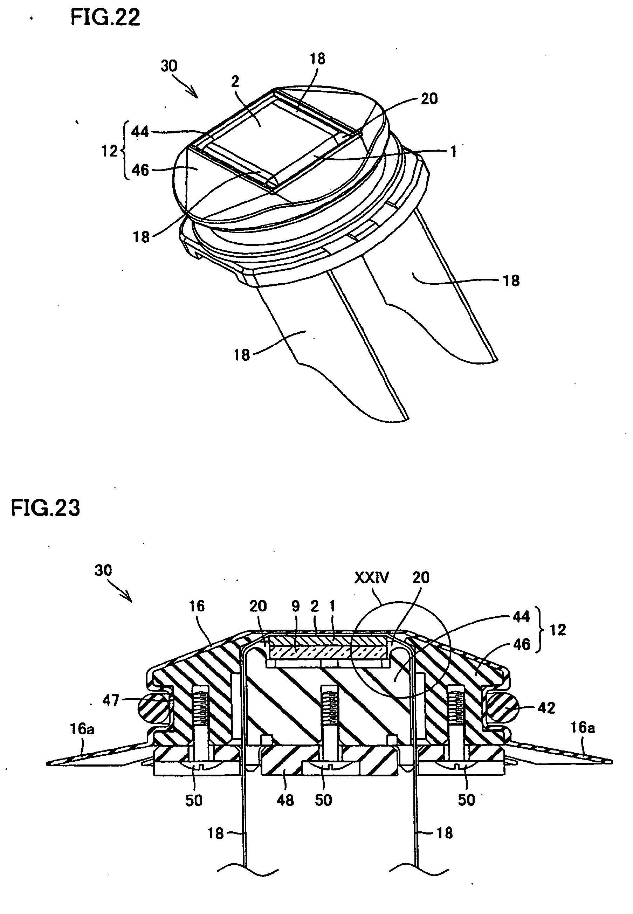

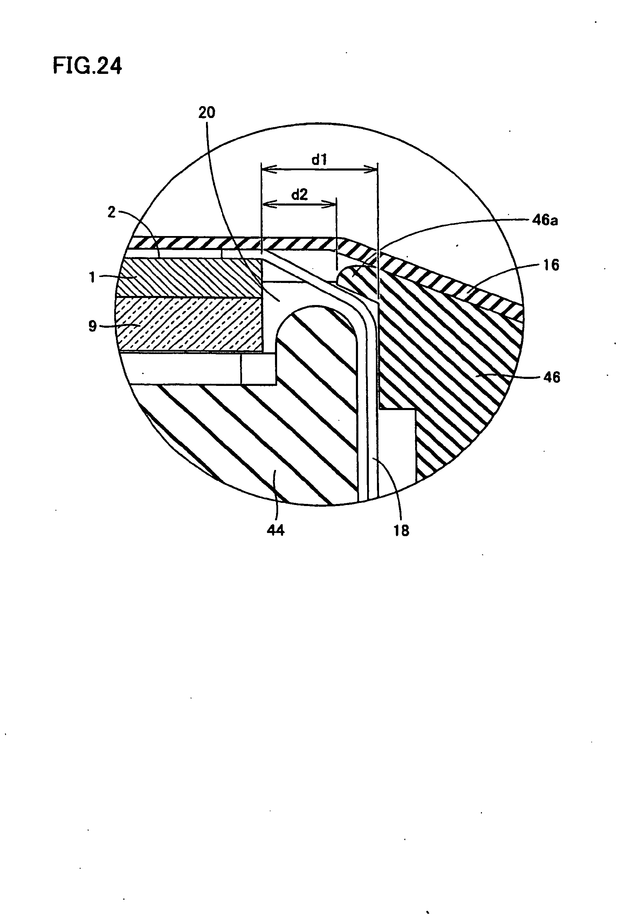

Initially, a structure of the pulse wave measuring apparatus in Embodiment 9 of the present invention will be described in detail. Fig. 21 is a schematic perspective view of a pressure-sensing portion of the pulse wave measuring apparatus in Embodiment 9 of the present invention. Fig. 22 is a schematic perspective view illustrating a state in which the protection film for the pressure-sensing portion shown in Fig. 21 is removed. Fig. 23 is a schematic cross-sectional view of the pressure-sensing portion shown in Fig. 21. Fig. 24 is an enlarged cross-sectional view of a region XXIV shown in Fig. 23. The pulse wave measuring apparatus in the present embodiment adopts a pressure-sensing device formed on the main surface of the semiconductor substrate as the pressure-sensing means, as in Embodiment 1 described above. It is noted that the same reference characters are given to components the same or corresponding to those in Embodiment 1 described above, and description thereof will not be repeated.

### (Structure of Pressure-Sensing Portion)

As shown in Figs. 21 to 23, pressure-sensing portion 30 of the pulse wave measuring apparatus in the present embodiment mainly includes semiconductor substrate 1 having the pressure-sensing device formed on the main surface, support member 9 supporting a back surface of semiconductor substrate 1, protection member 12 holding support member 9 and protecting semiconductor substrate 1, flexible line 18 electrically connected to semiconductor substrate 1, and protection film 16 attached to a contact portion with the body surface of pressure-sensing portion 30.

Protection member 12 includes a base portion 44 serving as an inner frame body containing the accommodation space and a cap portion 46 serving as an outer frame body fitted to base portion 44 so as to enclose an outer wall thereof. In other words, protection member 12 is divided into base portion 44 and cap portion 46.

Base portion 44 is shaped like a substantially rectangular parallelepiped, and has an accommodation space accommodating semiconductor substrate 1 and support member 9 in its upper portion. The accommodation space is formed by a concave portion provided in an upper surface of base portion 44. Cap portion 46 is formed such that its outer shape viewed from a direction orthogonal to the main surface of semiconductor substrate 1 disposed in the accommodation space is substantially circular. A concave fitting portion 47 fitting to the inner portion of O ring 42 serving as attachment means described later is provided in an outer circumferential wall of cap portion 46. Concave fitting portion 47 is located on the entire circumference of cap portion 46. A screw hole is provided on a lower surface of base portion 44 and cap portion 46. By attaching a screw 50 with an attachment plate 48 being interposed, base portion 44 and cap portion 46 are fixed.

In pressure-sensing portion 30 of the pulse wave measuring apparatus in the present embodiment, flexible line 18 having one end attached to the end portion of the main surface of semiconductor substrate 1 is inserted between base portion 44 and cap portion 46, and drawn out from the lower surface of pressure-sensing portion 30. Therefore, even after repeated use, coming off of the flexible line and detachment of protection film 16 is unlikely.

In addition, as shown in Fig. 24, cap portion 46 has an overhanging portion 46a facing, with a distance, a perimeter of the upper surface serving as an accommodation space forming surface of base portion 44. Overhanging portion 46a is provided so as to project from an inner surface of cap portion 46. Overhanging portion 46a is provided so as to cover a prescribed portion of flexible line 18 inserted between base portion 44 and cap portion 46 from above, and serves to protect flexible line 18 when pressure-sensing portion 30 is pressed against the living body.

In the present embodiment, a distance d1 between the end portion of semiconductor substrate 1 and the inner surface of cap portion 46 is adjusted to 1.4mm, and a distance d2 between the end portion of semiconductor substrate 1 and a tip end of overhanging portion 46a is adjusted to approximately 0.8mm. By setting distance d2 to not larger than 1.0mm as above, introduction of the skin in air chamber 20 when pressure-sensing portion 30 is pressed against the living body will be less likely. Accordingly, concentration on flexible line 18 of the pressurization force produced by pressing pressure-sensing portion 30 against the living body is avoided, and disconnection of flexible line 18 is prevented.

As shown in Figs. 21 and 23, protection film 16 is attached to cap portion 46 so as to cover the main surface of semiconductor substrate 1 and air chamber 20 located at the end portion of semiconductor substrate 1. Here, the peripheral portion of protection film 16 covers the outer circumferential wall of cap portion 46. By fitting the inner portion of O ring 42 around concave fitting portion 47 provided in the outer circumferential wall of cap portion 46 over protection film 16, protection film 16 is fastened and attached to cap portion 46. The outer portion of O ring 42 fitted to cap portion 46 is located outside concave fitting portion 47, and projects from the outer circumferential wall of cap portion 46. Here, protection film 16 is formed with a flexible member such as silicon rubber, and has a collar portion 16a extending in four directions provided in its peripheral portion. A cut portion 16b is provided between collar portions 16a.

As described above, protection film 16 is fixed by O ring 42, so that protection film 20 is unlikely to detach from cap portion 46 even after repeated use. In addition, O ring 42 is fitted to concave fitting portion 47 so that detachment of protection film 16 is further unlikely. Accordingly, a pulse wave measuring apparatus less likely to break after repeated use can be obtained.

In pressure-sensing portion 30 of the pulse wave measuring apparatus in the present embodiment, base portion 42 serving as the inner frame body and cap portion 44 serving as the outer frame body are formed with a ceramic material. If base portion 42 and cap portion 44 are formed with the ceramic material which is a material attaining high thermal conductivity, heat produced by current flow in the pressure-sensing device provided in semiconductor substrate 1 is effectively dissipated by base portion 42 and cap portion 44 through support member 9, which leads to suppression of temperature increase on the surface of pressure-sensing portion 30. Therefore, a pulse wave measuring apparatus less likely to cause cold burn and excellent in safety can be provided.

### (Assembly Procedure)

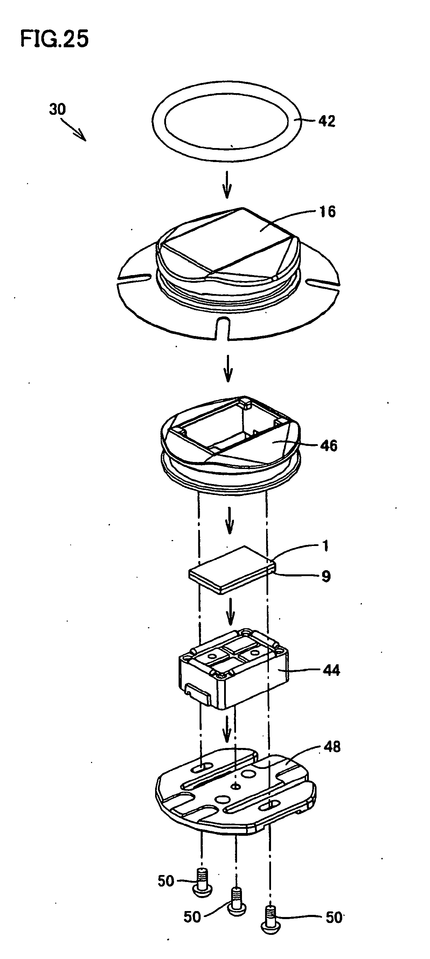

Fig. 25 is an exploded perspective view illustrating an assembly procedure of the pressure-sensing portion of the pulse wave measuring apparatus shown in Fig. 21. In the following, the assembly procedure of the pressure-sensing portion of the pulse wave measuring apparatus will be described with reference to Fig. 25.

First, support member 9 is joined to the back surface side of semiconductor substrate 1 having the flexible line (not shown) attached, by anodic bonding or the like. Successively, semiconductor substrate 1 joined to support member 9 is accommodated in the accommodation space provided in the upper portion of base portion 44, and fixed using an adhesive or the like.

Concurrently, protection film 16 covers cap portion 46, and is fixed to cap portion 46 by means of the O ring. Here, by grasping collar portion 16a provided in the peripheral portion of protection film 16, cap portion 46 can be covered with protection film 16 with excellent workability. In addition, as cut portion 16b is provided between collar portions 16a, workability is further improved.

Thereafter, cap portion 46 having protection film 16 attached is fitted to base portion 44 having semiconductor substrate 1 assembled. Then, attachment plate 48 is attached from below using screw 50. Here, the flexible line is drawn out of a slit provided in attachment plate 48.

As described above, assembly of pressure-sensing portion 30 as shown in Fig. 21 is completed. According to the pulse wave measuring apparatus having the structure described above, pressure-sensing portion 30 can be assembled with a very simple operation, and manufacturing cost can significantly be reduced.

### (Method of Measuring Output Characteristic)

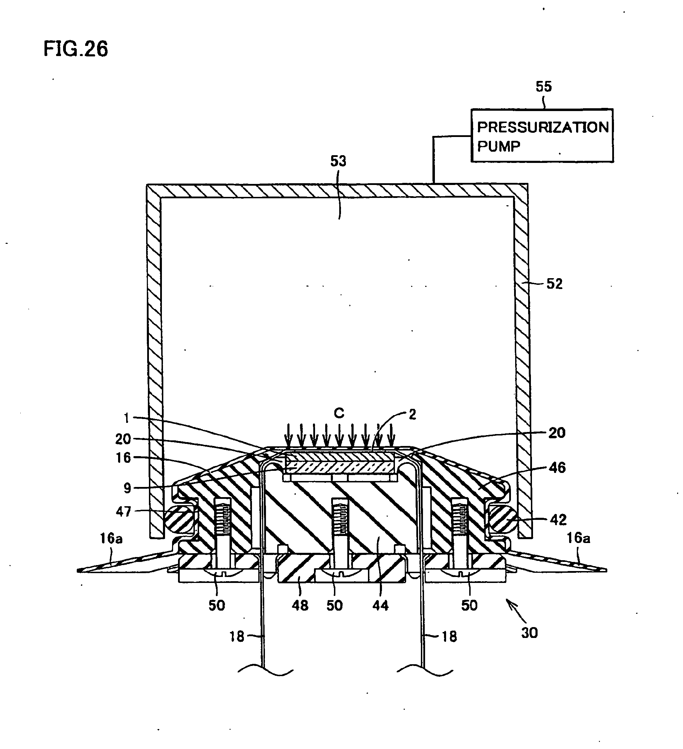

Fig. 26 is a schematic diagram illustrating a method of measuring an output characteristic of the pressure-sensing device in the pulse wave measuring apparatus in the present embodiment. In the pressure-sensing portion in the pulse wave measuring apparatus in the present embodiment, protection film 16 is fixed to cap portion 46 by means of O ring 42 as described above. By adopting such a structure, accurate and facilitated measurement of the output characteristic of the pressure-sensing device in the inspection process before product shipment can be carried out. A measurement method will be described in the following.

As shown in Fig. 26, a measurement jig 52 of a cylindrical shape with a bottom is prepared as a jig for measuring the output characteristic of the pressure-sensing device. Measurement jig 52 has a pressurization chamber 53 inside. Pressurization chamber 53 is connected to a pressurization pump 55, so that it can be pressurized by driving pressurization pump 55. An opening of measurement jig 52 is made so as to be larger than an outer shape of cap portion 46 of pressure-sensing portion 30 and so as to have an outer diameter equal to or slightly smaller than that of the O ring described above.

In order to measure the output characteristic of the pressure-sensing device formed in semiconductor substrate 1, it is necessary to uniformly apply a pressure on the entire surface of pressure-sensing surface 2 serving as the main surface of semiconductor substrate 1 as well as to monitor an output obtained from the pressure-sensing device. In the pulse wave measuring apparatus in the present embodiment, measurement of the output characteristic of the pressure-sensing device is carried out in such a manner that pressure-sensing portion 30 is covered with measurement jig 52 from above, the opening of measurement jig 52 is made to be in intimate contact with O ring 42, the pressure in pressurization chamber 53 is increased while maintaining the above-described state, and the output from the pressure-sensing device is monitored. By adopting such a method, the pressure by compressed air (a force shown with an arrow C in the figure) can uniformly be applied to the entire surface of pressure-sensing surface 2, so that accurate and rapid measurement of the output characteristic of the pressure-sensing device can be achieved.

### (Function and Effect)

As described above, by adopting the structure of the pressure-sensing portion as in the present embodiment, in addition to achieving the effect in Embodiment 1 described above, a pulse wave measuring apparatus free from disconnection of the flexible line and excellent in safety, in which the protection film is less likely to detach and the output characteristic of the pressure-sensing device is accurately and rapidly measured can be obtained. Therefore, a pulse wave measuring apparatus overcoming a variety of problems described above can be provided.

### (Variation)

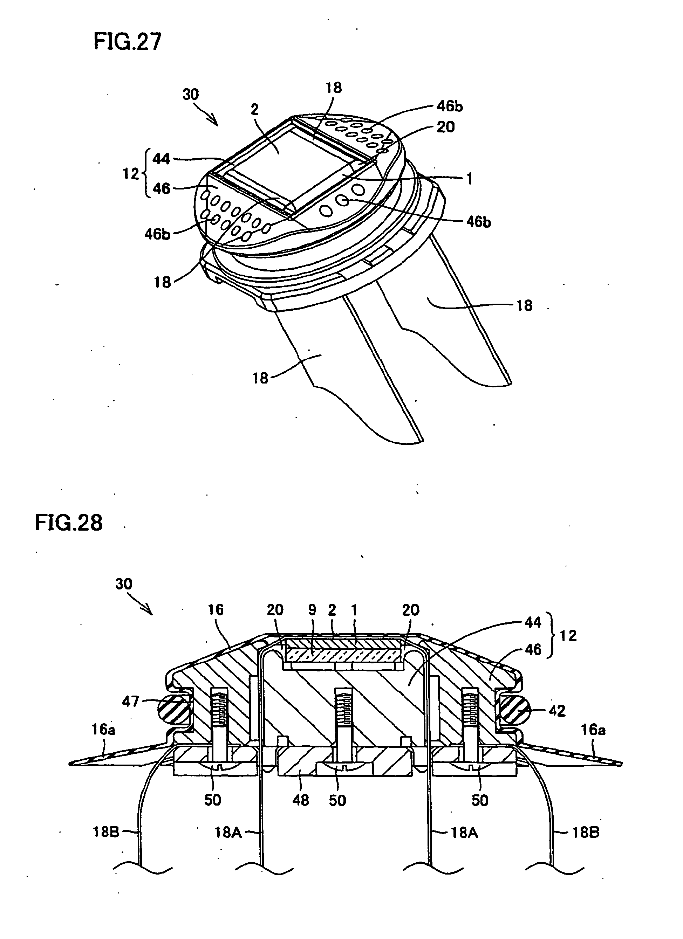

Fig. 27 is a schematic perspective view illustrating a state in which a protection film is removed, showing a variation of the pulse wave measuring apparatus in the present embodiment. As shown in Fig. 27, small irregularities are provided on an outer surface of cap portion 46, so that heat produced in semiconductor substrate 1 can further effectively be dissipated. Such irregularities are readily formed, for example, by providing a plurality of concave portions 46b on the outer surface of cap portion 46 as shown in Fig. 27. With such a structure, a surface area of cap portion 46 is increased, and heat dissipation performance is improved.

### (Embodiment 10)

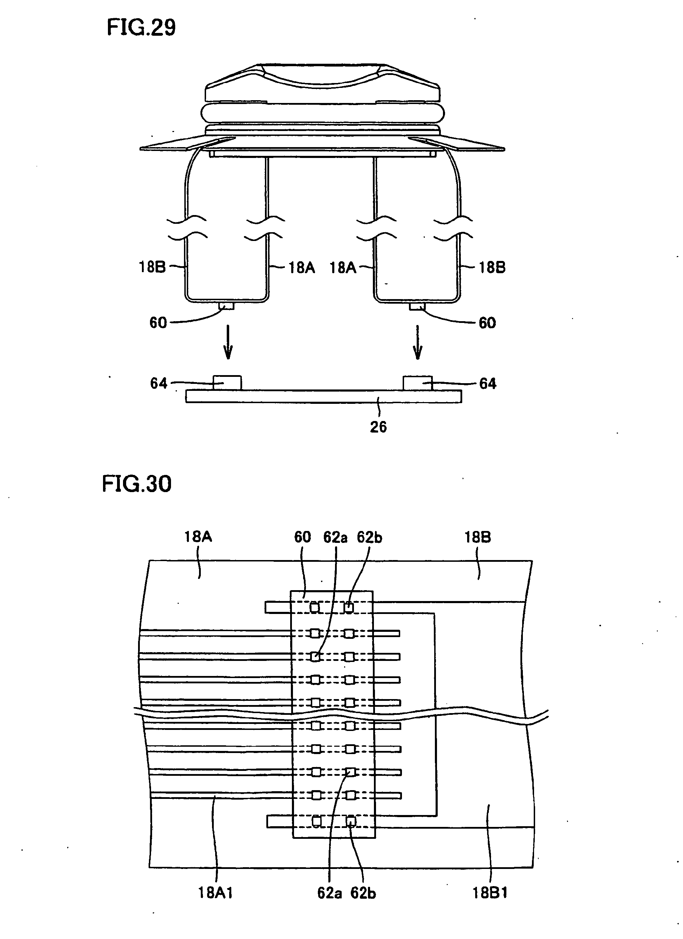

A structure of the pulse wave measuring apparatus in Embodiment 10 of the present invention will now be described in detail. Fig. 28 is a schematic cross-sectional view of a pressure-sensing portion of the pulse wave measuring apparatus in Embodiment 10 of the present invention. Fig. 29 is a schematic diagram illustrating a method of connection of the pressure-sensing portion shown in Fig. 28 to the circuit board. Fig. 30 is a plan view of a connector portion of the flexible line shown in Fig. 29. The pulse wave measuring apparatus in the present embodiment adopts a pressure-sensing device formed on the main surface of the semiconductor substrate as the pressure-sensing means, as in Embodiment 9 described above. It is noted that the same reference characters are given to components the same or corresponding to those in Embodiment 9 described above, and description thereof will not be repeated.

### (Structure of Pressure-Sensing Portion)

As shown in Fig. 28, pressure-sensing portion 30 of the pulse wave measuring apparatus in the present embodiment mainly includes semiconductor substrate 1 having the pressure-sensing device formed on the main surface, support member 9 supporting a back surface of semiconductor substrate 1, protection member 12 holding support member 9 and protecting semiconductor substrate 1, a flexible line 18A electrically connected to semiconductor substrate 1, a flexible line 18B electrically connected to protection member 12, and protection film 16 attached to a contact portion with the body surface of pressure-sensing portion 30.

Protection member 12, likewise in the pulse wave measuring apparatus shown in Embodiment 9 described above, includes base portion 44 serving as the inner frame body containing the accommodation space and cap portion 46 serving as the outer frame body fitted to base portion 44 so as to enclose the outer wall thereof. Base portion 44 and cap portion 46 are fixed by attachment plate 48 from below. The pulse wave measuring apparatus in the present embodiment, however, is different from the pulse wave measuring apparatus shown in Embodiment 9 described above in that base portion 44 and cap portion 46 are both formed of zinc which is a conductive material. In the pulse wave measuring apparatus in the present embodiment, base portion 44 and cap portion 46 are formed of zinc, considering formability and thermal conductivity. Meanwhile, any conductive material may be used for forming base portion 44 and cap portion 46, and noble metals (such as gold, silver, platinum, or the like), copper, aluminum, or the like may be employed, for example.

Pressure-sensing portion 30 of the pulse wave measuring apparatus in the present embodiment includes flexible line 18B having one end attached to cap portion 46 and attachment plate 48, in addition to flexible line 18A having one end attached to the end portion of semiconductor substrate 1. One end of flexible line 18B is clamped, for example, by cap portion 46 and attachment plate 48, so that flexible line 18B is electrically connected to cap portion 46 and attachment plate 48. Here, as attachment plate 48 abuts on base portion 44, base portion 44 is also electrically connected to flexible line 18B.

As shown in Fig. 29, connectors 60 are attached to flexible lines 18A and 18B drawn out of pressure-sensing portion 30 respectively. Though flexible lines 18A and 18B may be implemented by separate flexible lines, in the pulse wave measuring apparatus in the present embodiment, one flexible line is shared from a viewpoint of reduction in the number of parts and facilitated assembly operation. Specifically, flexible line 18 is connected such that one end thereof is electrically connected to the end portion of semiconductor substrate 1 and the other end thereof is electrically connected to cap portion 46 and attachment plate 48, and connector 60 is provided in a midpoint of flexible line 18.

As shown in Fig. 29, connector 60 provided in flexible line 18 is inserted in a socket 64 for establishing connection to circuit board 26. As shown in Fig. 30, flexible line 18 extending from the end portion attached to semiconductor substrate 1 includes a signal line 18A1 for the pressure-sensing device provided in semiconductor substrate 1, which is electrically connected to a connection pin 62a of connector 60. On the other hand, flexible line 18B extending from the end portion attached to cap portion 46 and attachment plate 48 is provided with a ground line 18B 1, which is electrically connected to a connection pin 62b of connector 60. Ground line 18B1 is electrically connected to an interconnection set to a ground potential and provided in circuit board 26, while connector 60 is attached to socket 64.

### (Function and Effect)

According to the structure as described above, base portion 44 and cap portion 46 are connected to the ground, so that base portion 44 and cap portion 46 serve as a lightning rod and electromagnetic shielding for the pressure-sensing device formed in semiconductor substrate 1. Accordingly, the pressure-sensing device is less susceptible to static electricity or noise from electric or magnetic field, and accurate and stable measurement of the pulse wave is allowed. According to the pulse wave measuring apparatus as in the present embodiment, a pulse wave measuring apparatus attaining excellent resistance to static electricity and noise from electric or magnetic field in addition to attaining the effect in Embodiment 9 described above can be obtained. In addition, as a conductive material generally has superior thermal conductivity, heat produced in semiconductor substrate 1 can effectively be dissipated to base portion 42 and cap portion 44.

If flexible line 18A having signal line 18A1 formed and flexible line 18B1 having ground line 18B1 formed are bundled by bringing them closer to each other such that ground line 18B1 faces signal line 18A1, superposition of the noise on the signal output from the pressure-sensing device is prevented, thereby attaining further accurate measurement of the pulse wave.

### (Embodiment 11)

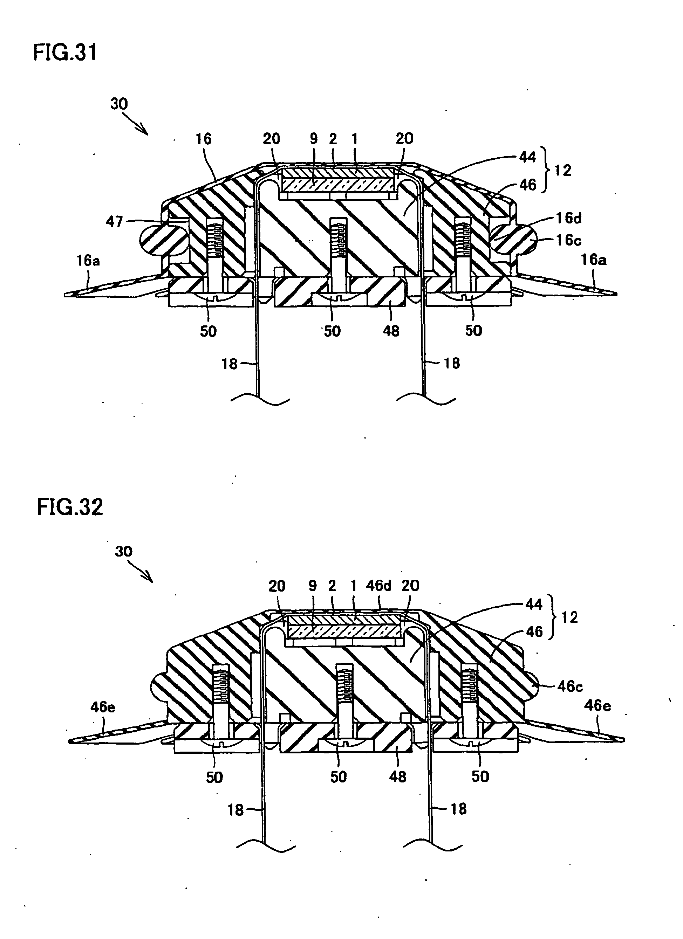

A structure of the pulse wave measuring apparatus in Embodiment 11 of the present invention will now be described in detail. Fig. 31 is a schematic cross-sectional view of a pressure-sensing portion of the pulse wave measuring apparatus in Embodiment 11 of the present invention. The pulse wave measuring apparatus in the present embodiment adopts a pressure-sensing device formed on the main surface of the semiconductor substrate as the pressure-sensing means, as in Embodiment 9 described above. It is noted that the same reference characters are given to components the same or corresponding to those in Embodiment 9 described above, and description thereof will not be repeated.

### (Structure of Pressure-Sensing Portion)

The pulse wave measuring apparatus in the present embodiment is implemented by integrally forming protection film 16 and O ring 42 in the pulse wave measuring apparatus in Embodiment 9 described above. Specifically, as shown in Fig. 31, a projected fitting portion 16d projecting toward inside and a projected portion 16c projecting toward outside are provided in a portion of protection film 16 facing the outer circumferential wall of cap portion 46, so as to integrally form protection film 16 and O ring 42 in Embodiment 9 described above.

Projected fitting portion 16d is provided in an area of protection film 16 around its entire periphery. Projected fitting portion 16d is fitted to concave fitting portion 47 provided on the outer circumferential wall of cap portion 46, so as to attach protection film 16 to cap portion 46. In other words, projected fitting portion 16d corresponds to the inner portion of O ring 42 in the pulse wave measuring apparatus shown in Embodiment 9 described above. Projected portion 16c is provided in an area of protection film 16 around its entire periphery, and serves as a portion being in intimate contact with the opening of the measurement jig used in measurement of the output characteristic of the pressure-sensing device in Embodiment 9 described above. That is, projected portion 16c corresponds to the outer portion of O ring 42 in the pulse wave measuring apparatus shown in Embodiment 9 described above.

### (Function and Effect)

According to the structure as above, a pulse wave measuring apparatus attaining facilitated assembly operation and reduction in manufacturing cost in addition to attaining the effect in Embodiment 9 described above can be obtained.

### (Embodiment 12)

A structure of the pulse wave measuring apparatus in Embodiment 12 of the present invention will now be described in detail. Fig. 32 is a schematic cross-sectional view of a pressure-sensing portion of a pulse wave measuring apparatus in Embodiment 12 of the present invention. The pulse wave measuring apparatus in the present embodiment adopts a pressure-sensing device formed on the main surface of the semiconductor substrate as the pressure-sensing means, as in Embodiment 9 described above. It is noted that the same reference characters are given to components the same or corresponding to those in Embodiment 9 described above, and description thereof will not be repeated.

### (Structure of Pressure-Sensing Portion)

The pulse wave measuring apparatus in the present embodiment is implemented by integrally forming protection film 16, O ring 42, and cap portion 46 in the pulse wave measuring apparatus in Embodiment 9 described above. Specifically, as shown in Fig. 32, a protection film portion 46d covering semiconductor substrate 1 and air chamber 20 is provided in the upper portion of cap portion 46, and a projected portion 46c projecting toward outside on the outer circumferential wall of cap portion 46 is provided, so as to integrally form protection film 16, O ring 42, and cap portion 46 in Embodiment 9 described above.

Projected portion 46c described above is provided on the entire outer circumferential wall of cap portion 46, and serves as a portion being in intimate contact with the opening of the measurement jig used in measurement of the output characteristic of the pressure-sensing device in Embodiment 9 described above. That is, projected portion 46c corresponds to the outer portion of O ring 42 in the pulse wave measuring apparatus shown in Embodiment 9 described above.

### (Function and Effect)

According to the structure as above, a pulse wave measuring apparatus attaining facilitated assembly operation and reduction in manufacturing cost in addition to attaining the effect in Embodiment 9 described above can be obtained. In addition, as the number of parts is further reduced as compared with the pulse wave measuring apparatus shown in Embodiment 11 described above, the assembly operation is further facilitated, and significant reduction in the manufacturing cost is achieved.

### (Other Variation)

In Embodiments 1 to 12 described above, though an example adopting a pressure-sensing device including a diaphragm as pressure-sensing means has been explained, the pressure-sensing means is not particularly limited thereto. For example, a strain gauge may be employed as the pressure-sensing means.

In addition, in Embodiments 1 to 12 described above, though an example in which the accommodation space accommodating the substrate is formed by providing a concave portion in the protection member has been explained, the accommodation space is not particularly limited thereto.

Moreover, in Embodiments 9 to 12 described above, though description has been provided on the basis of the pulse wave measuring apparatus having the air chamber formed at the end portion of the semiconductor substrate, such a basis is not essential. That is, any combination of the techniques disclosed in Embodiments 1 to 12 is possible, and any suitable combination can be adopted as necessary in accordance with conditions of use or the like.

Furthermore, though a pulse wave measuring apparatus measuring a pulse wave has exemplarily been described in Embodiments 1 to 12 as above, the present invention is applicable to any apparatus for measuring a contact pressure with respect to the body surface by pressurization against the body surface, such as an ocular tension measuring apparatus.

### Industrial Applicability

The present invention is utilized in a pressurization-type pulse wave measuring apparatus measuring data of a living body in a non-invasive manner in order to know medical condition of a subject.

## Claims

1. A pulse wave measuring apparatus comprising:
a substrate (1) having pressure-sensing means (3) on a main surface; and
a protection member (12) having an accommodation space accommodating said substrate (1), the pulse wave measuring apparatus serving to measure a pulse wave by pressing said substrate (1) against a living body;
said protection member (12) is formed of a conductive material,
**characterized in that** a wall surface (20a) of said protection member (12) forming said accommodation space is arranged such that an air chamber (20) is interposed between said wall surface and an end surface of said substrate (1), and
said air chamber (20) is open to atmosphere.

2. The pulse wave measuring apparatus according to claim 1, wherein
said air chamber (20) is provided around an entire perimeter of said substrate (1).

3. The pulse wave measuring apparatus according to claim 1, further comprising a protection film (16) covering said main surface of said substrate (1) and said air chamber (20), wherein
said protection member (12) has a communication hole (13, 14) such that said air chamber (20) is open to atmosphere.

4. The pulse wave measuring apparatus according to claim 1, further comprising a circuit board (26) processing a signal, and a flexible line (18) transmitting a signal output from said pressure-sensing means (3) to said circuit board (26), wherein
said flexible line (18) includes a fixed portion (18a) fixed to said protection member (12), a connection portion (18b) connected to said substrate (1), and a loosened portion (18c) located between said fixed portion (18a) and said connection portion (18b).

5. The pulse wave measuring apparatus according to claim 4, wherein
said loosened portion (18c) is located inside said air chamber (20).

6. The pulse wave measuring apparatus according to claim 1, further comprising a circuit board (26) processing a signal, and a flexible line (18) transmitting a signal output from said pressure-sensing means (3) to said circuit board (26), wherein
said flexible line (18) includes a fixed portion (18a) fixed to said protection member (12) and a connection portion (18b) connected to said substrate (1), and
a portion (18d) having rigidity different from that of another portion of said flexible line (18) is located between said fixed portion (18a) and said connection portion (18c) of said flexible line (18).

7. The pulse wave measuring apparatus according to claim 1, further comprising
a protection film (16) covering said main surface of said substrate (1) and said air chamber (20), and
attachment means (42) for fastening a peripheral portion of said protection film (16) to an outer circumferential wall of said protection member (12) for attachment.

8. The pulse wave measuring apparatus according to claim 7, wherein
said protection member (12) has a substantially circular outer shape when viewed from a direction orthogonal to said main surface of said substrate (1), and said attachment means (42) is an O ring.

9. The pulse wave measuring apparatus according to claim 8, wherein
said outer circumferential wall of said protection member (12) has a concave fitting portion (47) fitting to an inner portion of said O ring (42) on an entire circumference, and
an outer portion of said O ring (42) projects from said outer circumferential wall of said protection member (12).

10. The pulse wave measuring apparatus according to claim 7, wherein
said protection film (16) and said attachment means (42) are integrally formed.

11. The pulse wave measuring apparatus according to claim 7, wherein
said protection film (16) has a collar portion (16a) in said peripheral portion.

12. The pulse wave measuring apparatus according to claim 1, wherein
said protection member (12) includes an inner frame body (44) containing said accommodation space and an outer frame body (46) fitted to said inner frame body (44) so as to enclose an outer wall of said inner frame body (44),
said outer frame body (46) has a protection film portion (46d) covering said main surface of said substrate (1) and said air chamber (20), and
an outer circumferential wall of said outer frame body (46) has a projected portion (46c) on its entire circumference.

13. The pulse wave measuring apparatus according to claim 1, further comprising a circuit board (26) processing a signal, and a flexible line (18) transmitting a signal output from said pressure-sensing means (3) to said circuit board (26), wherein
said protection member (12) includes an inner frame body (44) containing said accommodation space and an outer frame body (46) fitted to said inner frame body (44) so as to enclose an outer wall of said inner frame body (44), and
said flexible line (18) is inserted between said inner frame body (44) and said outer frame body (46).

14. The pulse wave measuring apparatus according to claim 13, wherein
said outer frame body (46) has an overhanging portion (46a) provided so as to project from an inner surface of said outer frame body (46) and facing, with a distance, a perimeter of an accommodation space forming surface of said inner frame body (44) where said accommodation space is formed, and
said flexible line (18) inserted between said inner frame body (44) and said outer frame body (46) is protected by said overhanging portion (46a).

15. The pulse wave measuring apparatus according to claim 1, wherein
said protection member (12) is electrically connected to a ground potential.

16. The pulse wave measuring apparatus according to claim 15, further comprising a circuit board (26) processing a signal, and a flexible line (18) transmitting a signal output from said pressure-sensing means (3) to said circuit board (26), wherein
said protection member (12) is electrically connected to the ground potential by means of said flexible line (18).

17. The pulse wave measuring apparatus according to claim 1, wherein
said protection member (12) is formed with a metal material or a ceramic material.

18. The pulse wave measuring apparatus according to claim 1, wherein
said protection member (12) has a plurality of small irregularities on its surface.

## Patentansprüche

1. Pulswellenmessvorrichtung, welche aufweist:
ein Substrat (1) mit Druckfühlmitteln (3) auf einer Hauptfläche; und
ein Schutzelement (12) mit einem Aufnahmeraum zur Aufnahme des Substrats (1), wobei die Pulswellenmessvorrichtung dazu dient, eine Pulswelle durch Drücken des Substrats (1) gegen einen lebenden Körper zu messen;
wobei das Schutzelement (12) aus einem leitfähigen Material ausgebildet ist,
**dadurch gekennzeichnet, dass** eine Wandfläche (20a) des den Aufnahmeraum bildenden Schutzelements (12) so eingerichtet ist, dass eine Luftkammer (20) zwischen der Wandfläche und einer Endfläche des Substrats (1) zwischengelegt ist, und
die Luftkammer (20) zur Atmosphäre hin offen ist.

2. Pulswellenmessvorrichtung nach Anspruch 1, wobei
die Luftkammer (20) um den gesamten Umfang des Substrats (1) herum vorgesehen ist.

3. Pulswellenmessvorrichtung nach Anspruch 1, welche ferner einen die Hauptfläche des Substrats (1) und die Luftkammer (20) abdeckenden Schutzfilm (16) aufweist, wobei
das Schutzelement (12) ein Kommunikationsloch (13, 14) derart aufweist, dass die Luftkammer (20) zur Atmosphäre hin offen ist.

4. Pulswellenmessvorrichtung nach Anspruch 1, welcher ferner eine ein Signal verarbeitende Leiterplatte (26) und eine flexible Leitung (18), welche ein von den Druckfühlmitteln (3) ausgegebenes Signal auf die Leiterplatte (26) überträgt, aufweist, wobei
die flexible Leitung (18) einen an der Schutzkammer (12) befestigten festen Abschnitt (18a), einen mit dem Substrat (1) verbundenen Verbindungsabschnitt (18b) und einen lockeren Abschnitt (18c), der zwischen dem festen Abschnitt (18a) und dem Verbindungsabschnitt (18b) angeordnet ist, enthält.

5. Pulswellenmessvorrichtung nach Anspruch 4, wobei
der lockere Abschnitt (18c) innerhalb der Luftkammer (20) angeordnet ist.

6. Pulswellenmessvorrichtung nach Anspruch 1, welche ferner eine ein Signal verarbeitende Leiterplatte (26) und eine flexible Leitung (18), die ein von den Druckfühlmitteln (3) ausgegebenes Signal auf die Leiterplatte (26) überträgt, aufweist, wobei
die flexible Leitung (18) einen an dem Schutzelement (12) befestigten festen Abschnitt (18a) und einen mit dem Substrat (1) verbundenen Verbindungsabschnitt (18b) enthält, und
ein Abschnitt (18d), welcher eine von derjenigen eines weiteren Abschnitts der flexiblen Leitung (18) verschiedene Steifigkeit hat, zwischen dem festen Abschnitt (18a) und dem Verbindungsabschnitt (18c), der flexiblen Leitung (18) angeordnet ist.

7. Pulswellenmessvorrichtung nach Anspruch 1, welcher ferner aufweist:
einen die Hauptfläche des Substrats (1) und die Luftkammer (20) abdeckenden Schutzfilm (16), und
Anbringungsmittel (42) zur Befestigung eines Randabschnitts des Schutzfilms (16) an einer Umfangswand des Schutzelements (12) zur Anbringung.

8. Pulswellenmessvorrichtung nach Anspruch 7, wobei
das Schutzelement (12) eine bei Blick aus einer Richtung senkrecht zu der Hauptfläche des Substrats (1) im Wesentlichen kreisförmige äußere Form aufweist und die Anbringungsmittel (42) ein O-Ring sind.

9. Pulswellenmessvorrichtung nach Anspruch 8, wobei
die äußere Umfangswand des Schutzelements (12) einen konkaven Passabschnitt (47) aufweist, der auf einem gesamten Umfang zu einem inneren Abschnitt des O-Rings (42) passt, und
ein äußerer Abschnitt des O-Rings (42) aus der äußeren Umfangswand des Schutzelements (12) herausragt.

10. Pulswellenmessvorrichtung nach Anspruch 7, wobei
der Schutzfilm (16) und die Anbringungsmittel (42) integriert ausgebildet sind.

11. Pulswellenmessvorrichtung nach Anspruch 7, wobei
der Schutzfilm (16) einen Bundabschnitt (16a) in dem Randabschnitt aufweist.

12. Pulswellenmessvorrichtung nach Anspruch 1, wobei
das Schutzelement (12) einen inneren Rahmenkörper (44), der den Aufnahmeraum enthält, und einen äußeren Rahmenkörper (46), der an den inneren Rahmenkörper (44) so angepasst ist, dass er eine Außenwand des inneren Rahmenkörpers (44) umschließt, enthält,
der äußere Rahmenkörper (46) einen Schutzfilmabschnitt (46d) aufweist, der die Hauptfläche des Substrats (1) und die Luftkammer (20) abdeckt, und
eine äußere Umfangswand des äußeren Rahmenkörpers (46) einen vorspringenden Abschnitt (46c) an ihrem gesamten Umfang aufweist.

13. Pulswellenmessvorrichtung nach Anspruch 1, welche ferner eine ein Signal verarbeitende Leiterplatte (26) und eine flexible Leitung (18) aufweist, welche ein von den Druckfühlmitteln (3) ausgegebenes Signal zu der Leiterplatte (26) überträgt, wobei
das Schutzelement (12) einen den Aufnahmeraum enthaltenden inneren Rahmenkörper (44) und einen an den inneren Rahmenkörper (44) angepassten äußeren Rahmenkörper (46) so aufweist, dass eine Außenwand des inneren Rahmenkörpers (44) umschlossen wird, und
die flexible Leitung (18) zwischen dem inneren Rahmenkörper (44) und dem äußeren Rahmenkörper (46) eingefügt ist.

14. Pulswellenmessvorrichtung nach Anspruch 13, wobei
der äußere Rahmenkörper (46) einen überhängenden Abschnitt (46a) aufweist, der so vorgesehen ist, dass er von einer Innenfläche des äußeren Rahmenkörpers (46) abragt und mit Abstand einem Umfang einer einen Aufnahmeraum bildenden Fläche des inneren Rahmenkörpers (44), wo der Aufnahmeraum ausgebildet ist, zugekehrt ist, und
die zwischen dem inneren Rahmenkörper (44) und dem äußeren Rahmenkörper (46) eingefügte flexible Leitung (18) durch den überhängenden Abschnitt (46a) geschützt ist.

15. Pulswellenmessvorrichtung nach Anspruch 1, wobei
das Schutzelement (12) elektrisch mit Massepotential verbunden ist.

16. Pulswellenmessvorrichtung nach Anspruch 15, welche ferner eine ein Signal verarbeitende Leiterplatte (26) und eine flexible Leitung (18) aufweist, die ein von den Druckfühlmitteln (3) ausgegebenes Signal zu der Leiterplatte (26) überträgt, wobei
das Schutzelement (12) elektrisch mit dem Massepotential mittels der flexiblen Leitung (18) verbunden ist.

17. Pulswellenmessvorrichtung nach Anspruch 1, wobei
das Schutzelement (12) mit einem Metallmaterial oder einem Keramikmaterial ausgebildet ist.

18. Pulswellenmessvorrichtung nach Anspruch 1, wobei
das Schutzelement (12) eine Anzahl kleiner Unregelmäßigkeiten auf seiner Oberfläche aufweist.

## Revendications

1. Appareil de mesure d'une onde de pression comprenant :
un substrat (1) qui présente des moyens de détection de pression (3) sur une surface principale ; et
un élément de protection (12) qui présente un espace de réception qui reçoit ledit substrat (1), l'appareil de mesure d'une onde de pression servant à mesurer une onde de pression en pressant ledit substrat (1) contre un corps vivant ;
ledit élément de protection (12) est réalisé dans un matériau conducteur ;
**caractérisé en ce que** :
une surface de paroi (20a) dudit élément de protection (12) qui forme ledit espace de réception est agencée de telle sorte qu'une chambre d'air (20) soit interposée entre ladite surface de paroi et une surface d'extrémité dudit substrat (1) ; et
ladite chambre d'air dite (20) est ouverte à l'atmosphère.

2. Appareil de mesure d'une onde de pression selon la revendication 1, dans lequel :
ladite chambre d'air (20) est disposée autour d'un périmètre entier dudit substrat(1).

3. Appareil de mesure d'une onde de pression selon la revendication 1, comprenant en outre un film de protection (16) qui couvre ladite surface principale dudit substrat (1) et ladite chambre d'air (20), dans lequel :
ledit élément de protection (12) présente un trou de communication (13, 14) de telle sorte que ladite chambre d'air (20) soit ouverte à l'atmosphère.

4. Appareil de mesure d'une onde de pression selon la revendication 1, comprenant en outre une carte de circuit (26) qui traite un signal, et une ligne flexible (18) qui transmet une sortie de signal en provenance desdits moyens de détection de pression (3) à ladite carte (26), dans lequel :
ladite ligne flexible (18) comprend une partie fixe (18a) fixée audit élément de protection (12), une partie de connexion (18b) connectée audit substrat (1), et une partie lâche (18c) qui se situe entre ladite partie fixe (18a) et ladite partie de connexion (18b).

5. Appareil de mesure d'une onde de pression selon la revendication 4, dans lequel :
ladite partie lâche (18c) se situe à l'intérieur de ladite chambre d'air (20).

6. Appareil de mesure d'une onde de pression selon la revendication 1, comprenant en outre une carte de circuit (26) qui traite un signal, et une ligne flexible (18) qui transmet une sortie de signal en provenance desdits moyens de détection de pression (3) à ladite carte de circuit (26), dans lequel :
ladite ligne flexible (18) comprend une partie fixe (18a) fixée audit élément de protection (12) et une partie de connexion (18b) connectée audit substrat (1) ; et
une partie (18d) qui présente une rigidité différente de celle d'une autre partie de ladite ligne flexible (18), se situe entre ladite partie fixe (18a) et ladite partie de connexion (18c) de ladite ligne flexible (18).

7. Appareil de mesure d'une onde de pression selon la revendication 1, comprenant en outre :
un film de protection (16) qui couvre ladite surface principale dudit substrat (1) et ladite chambre d'air (20) ; et
des moyens de fixation (42) destinés à fixer une partie périphérique dudit film de protection (16) à une paroi circonférentielle extérieure dudit élément de protection (12) pour une fixation.

8. Appareil de mesure d'une onde de pression selon la revendication 7, dans lequel :
ledit élément de protection (12) présente une forme extérieure sensiblement circulaire quand on regarde à partir d'une direction orthogonale par rapport à ladite surface principale dudit substrat (1), et lesdits moyens de fixation (42) sont un joint torique.

9. Appareil de mesure d'une onde de pression selon la revendication 8, dans lequel :
ladite paroi circonférentielle extérieure dudit élément de protection (12) présente une partie d'ajustage concave (47) qui s'ajuste à une partie intérieure dudit joint torique (42) sur une circonférence entière ; et
une partie extérieure dudit joint torique (42) fait saillie à partir de ladite paroi circonférentielle extérieure dudit élément de protection (12).

10. Appareil de mesure d'une onde de pression selon la revendication 7, dans lequel :
ledit film de protection (16) et lesdits moyens de fixation (42) sont formés d'une pièce.

11. Appareil de mesure d'une onde de pression selon la revendication 7, dans lequel:
ledit film de protection (16) présente une partie de collier (16a) dans ladite partie périphérique.

12. Appareil de mesure d'une onde de pression selon la revendication 1, dans lequel :
ledit élément de protection (12) comprend un corps de cadre intérieur (44) qui contient ledit espace de réception et un corps de cadre extérieur (46) ajusté audit corps de cadre intérieur (44) de façon à enfermer une paroi extérieure dudit corps de cadre intérieur (44) ;
ledit corps de cadre extérieur (46) présente une partie de film de protection (46d) qui couvre ladite surface principale dudit substrat (1) et ladite chambre d'air (20) ; et
une paroi circonférentielle extérieure dudit corps de cadre extérieur (46) présente une partie qui fait saillie (46c) sur sa circonférence entière.

13. Appareil de mesure d'une onde de pression selon la revendication 1, comprenant en outre une carte de circuit (26) qui traite un signal, et une ligne flexible (18) qui transmet une sortie de signal en provenance desdits moyens de détection de pression (3) à ladite carte de circuit (26), dans lequel :
ledit élément de protection (12) comprend un corps de cadre intérieur (44) qui contient ledit espace de réception et un corps de cadre extérieur (46) ajusté audit corps de cadre intérieur (44) de façon à enfermer une paroi extérieure dudit corps de cadre intérieur (44) ; et
ladite ligne flexible (18) est insérée entre ledit corps de cadre intérieur (44) et ledit corps de cadre extérieur (46).

14. Appareil de mesure d'une onde de pression selon la revendication 13, dans lequel :
ledit corps de cadre extérieur (46) présente une partie en surplomb (46a) disposée de façon à faire saillie à partir d'une surface intérieure dudit corps de cadre extérieur (46) et qui fait face, à une distance, à un périmètre d'une surface qui forme un espace de réception dudit corps de cadre intérieur (44) où est formé ledit espace de réception ; et
ladite ligne flexible (18) insérée entre ledit corps de cadre intérieur (44) et ledit corps de cadre extérieur (46) est protégée par ladite partie en surplomb (46a).

15. Appareil de mesure d'une onde de pression selon la revendication 1, dans lequel :
ledit élément de protection (12) est connecté de manière électrique au potentiel de la masse.

16. Appareil de mesure d'une onde de pression selon la revendication 15, comprenant en outre une carte de circuit (26) qui traite un signal, et une ligne flexible (18) qui transmet une sortie de signal en provenance desdits moyens de détection de pression (3) à ladite carte (26), dans lequel :
ledit élément de protection (12) est connecté de manière électrique au potentiel de la masse au moyen de ladite ligne flexible (18).

17. Appareil de mesure d'une onde de pression selon la revendication 1, dans lequel :
ledit élément de protection (12) est réalisé dans un matériau métallique ou dans un matériau céramique.

18. Appareil de mesure d'une onde de pression selon la revendication 1, dans lequel :
ledit élément de protection (12) présente une pluralité de petites irrégularités situées sur sa surface.
